# EUROPEAN PATENT SPECIFICATION

(11) **EP 1 002 068 B1**
(45) Date of publication and mention of the grant of the patent: **09.09.2009**
(21) Application number: 98935228.1
(22) Date of filing: 14.07.1998
(51) Int. Cl.: C12N 15/00, C12N 15/12, C07K 14/495, C12N 5/10, C12Q 1/68, A01K 67/027, A61K 48/00

(54) **MUTATIONS IN THE MYOSTATIN GENE CAUSE DOUBLE-MUSCLING IN MAMMALS**
MUTATIONEN IM MYOSTATINGEN STEIGERN MUSKELMASSE IN SÄUGETIEREN
MUTATION DU GENE DE LA MYOSTATINE A L'ORIGINE DE L'HYPERTROPHIE MUSCULAIRE CHEZ LES MAMMIFERES

(30) Priority: 14.07.1997 US 891789; 15.01.1998 US 7761
(43) Date of publication of application: 24.05.2000
(60) Divisional application: 08006554.3
(73) Proprietor: University of Liège, 4000 Liège (BE)
(72) Inventor: GROBET, Luc, B-4130 Esneux (BE); GEORGES, Michel, B-4161 Villers-aux-Tours (BE); PONCELET, Dominique, B-3700 Tongeren (BE)
(74) Representative: Vossius & Partner
(86) International application number: PCT/IB1998/001197
(87) International publication number: WO 1999/002667

(56) References cited:
- WO-A-94/21681
- WO-A-98/33887
- MCPHERRON, LAWLER AND LEE: "Regulation of skeletal muscle mass in mice by a new TGF-beta superfamily member." NATURE, vol. 387, 1 May 1997, pages 83-90, XP002085797 cited in the application
- CHARLIER ET AL.: "The mh gene causing double-muscling in cattle maps to the bovine chromosome 2." MAMMALIAN GENOME, vol. 6, no. 11, 1995, pages 788-792, XP002085798 cited in the application
- GROBET ET AL.: "Molecular definition of an allelic series of mutations disrupting the myostatin function and causing double-muscling in cattle" MAMM. GENOME, vol. 9, no. 3, March 1998, pages 210-213, XP002085799
- GROBET ET AL.: "A deletion in the bovine myostatin gene causes the double-muscled phenotype in cattle." NATURE GENETICS, vol. 17, no. 1, September 1997, pages 71-74, XP002085800 & WESTHUSIN, M.: "From mighty mice to mighty cows." NATURE GENETICS, vol. 17, no. 1, September 1997, pages 4-5,
- MCPHERRON AND LEE: "Double muscling in cattle due to mutations in the myostatin gene" PROC. NATL. ACAD. SCI. USA, vol. 94, no. 23, 11 November 1997, pages 12457-12461, XP002085801
- KAMBADUR ET AL.: "Mutations in myostatin (GDF8) in double-muscled Belgian Blue and Piedmontese cattle." GENOME RESEARCH, vol. 7, no. 9, September 1997, pages 910-916, XP002085802
- SMITH ET AL.: "Myostatin maps to the interval containing the bovine mh locus." MAMMALIAN GENOME, vol. 8, no. 10, October 1997, pages 742-744, XP002085803
- DICKMAN: "Gene mutation provides more meat on the hoof." SCIENCE, vol. 277, no. 5334, 26 September 1997, pages 1922-1923, XP002085804
- WESTHUSIN. M.: "For mighty mice to mighty cows" NATURE GENETICS, vol. 17, no. 1, September 1997, pages 71-74, XP002086548
- GEORGES AND ANDERSSON: "Livestock genomics comes of age" GENOME RESEARCH, vol. 6, 1996, pages 907-921, XP002085805 cited in the application
- KAPPES ET AL.: "A second-generation linkage map of the bovine genome" GENOME RESEARCH, vol. 7, 1997, pages 235-249, XP002085806 cited in the application

## Description

### Field of The Invention

This invention relates to factors affecting muscle development in mammals, especially livestock. In particular, this invention relates to transgenic male non-human mammals containing a transgene encoding a mutated myostatin protein located on the Y chromosome having a phenotype characterized by muscular hyperplasia and a method for creating a cell for use in creating the transgenic mammal.

### Description Of Related Art

The TGF-β superfamily consists of a group of multifunctional polypeptides which control a wide range of differentiation processes in many mammalian cell types. GDF-8 is a member of the TGF-β superfamily. All members of this superfamily share a common structure including a short peptide signal for secretion and an N-terminal peptide fragment that is separated from the bioactive carboxy-terminal fragment by proteolytic cleavage at a highly conserved proteolytic cleavage site. The bioactive carboxy-terminal domain is characterized by cysteine residues at highly conserved positions which are involved in intra- and intermolecular disulfide bridges. The functional molecules are covalently linked (via a S-S bond) dimers of the carboxy-terminal domain (Masterson *et al*., 1996).

Recently, it was reported that mice deficient in the gene coding for GDF-8 were characterized by a generalized muscular hyperplasia (McPherron *et al*., 1997). The GDF-8 deficient mice were produced by gene targeting using homologous recombination in embryonic stem cells, a method referred to as "gene knock-out". The murine generalized muscular hyperplasia appeared to be very similar in its expression to the muscular hyperplasia characterizing "double-muscled" cattle. This observation raised the intriguing possibility that the bovine gene coding for GDF-8 (i.e. the bovine evolutionary homologue of the mouse GDF-8 gene) is involved in the bovine double-muscling phenotype. It also raised the possibility that the human gene coding for GDF-8 (i.e. the human evolutionary homologue of the mouse GDF-8 gene) is involved in regulating muscular development in humans, specifically skeletal muscle genesis. Isolation of the human GDF-8 gene may have therapeutic uses/apptications in the treatment of musculodegenerative diseases through upgrading or downgrading the expression of GDF-8.

The occurrence of animals characterized by a distinct generalized muscular hypertrophy, commonly known as "double-muscled" animals, has been reported in several cattle breeds around the world. The first documented description of double-muscled cattle dates back as early as 1807 (Culley, 1807). One of the breeds in which this characteristic has been most thoroughly analyzed is the Belgian Blue Cattle Breed ("Belgian Blue Breed"). This is one of the only breeds where the double-muscled trait has been systematically selected for, and where the double-muscled phenotype is virtually fixed. A comparison of double-muscled and conventional animals within the Belgian Blue Breed, showed an increase in muscle mass by 20% on average, while all other organs were reduced in size (Hanset, 1986 and 1991). The muscular hypertrophy was shown to be an histological hyperplasia affecting primarily superficial muscles, accompanied by a 50% reduction in total lipid content and a reduction in connective tissue fraction as measured by hydroxyproline content (Hanset *et al*., 1982). Double-muscled animals were shown to have a reduced feed intake with improved feed conversion ratio (Hanset *et al*., 1987). An important economic benefit of double-muscled animals, in contrast to conventional animals, is the substantial increase in selling price and net income for the farmer (Hanset *et al*., 1987).

One of the most thorough series of studies on double-muscling is that of Hanset and colleagues in the Belgian Blue Breed. Objective criteria of muscular development, such as dressing-out percentage, lean and fat percentage, plasma and red cell creatine and creatinine concentrations, were measured on nearly 150 randomly selected animals raised in standardized conditions. These studies clearly revealed abnormal, bimodal distributions of the double-muscled phenotype and objectively confirmed the visual classification traditionally performed by breeders on double-muscled and conventional animals. The phenotypic distribution was resolved using a maximum likelihood procedure into two component normal populations with a common variance which revealed mean differences of three to four standard deviations depending on the trait. This suggested the presence of an allele having a major effect on muscular development with a population frequency close to 50% (Hanset and Michaux, 1985b). The most convincing evidence in favour of such an allele, however, came from experimental crosses involving double-muscled Belgian Blue sires and Holstein Friesian dairy cows (the latter animals having very poor muscular development). While F1 offspring showed a phenotypic distribution very similar to their Holstein Friesian dams, backcrossing these F1's to double-muscled sires produced a bimodal BC generation, clearly pointing towards the Mendelian segregation of a recessive "*mh*" (muscular hypertrophy) allele (Hanset and Michaux., 1985a).

The same kind of experimental crosses were subsequently used to perform a whole genome scan using a microsatellite based marker map. To perform the linkage analysis, animals were classified as double-muscled or conventional. Very significant Logarithm of the Odds scores (lodscores) were obtained on chromosome 2 (> 17), and multi point linkage analysis positioned the *mh* locus at the centromeric end of this chromosome, at [2]centimorgan from the nearest microsatellite marker: TGLA44. The corresponding chromosomal region accounted for all the variance of the trait assumed to be fully penetrant in this experiment (Charlier *et al*., 1995).

In humans, genes coding for some forms of muscular abnormalities have been isolated, e.g. muscular dystrophy. The present invention provides for the gene which regulates the development of skeletal muscle only, as opposed to other types of muscle, e.g. smooth or cardiac muscle. The present invention may provide an understanding of the role of the GDF-8 gene or its receptor in the regrowth of skeletal muscle in humans which only undergo a hyperplasic response.

### Summary of the Invention

The present inventors have identified and sequenced a gene (cDNA and genomic) encoding a bovine myostatin protein. The nucleic acid coding sequence is identified as SEQ ID NO:1 and the protein sequence is identified as SEQ ID NO:2. The genomic bovine sequence is identified as SEQ ID NO:54. A mutant gene (SEQ ID NO:3) in which the coding sequence lacks an 11-base pair consecutive sequence (SEQ ID NQ:11) of the sequence encoding bovine protein having myostatin activity has been sequenced. It has been shown that cattle of the Belgian Blue breed homozygous for the mutant gene lacking myostatin activity are double-muscled. Other bovine mutations which lead to double-muscling in have also been determined, being identified herein as *nt419(del7-ins10)*, *Q204X, E226X and C313Y*, respectively.

Described is a method for determining the presence of muscular hyperplasia in a mammal. The method includes obtaining a sample of material containing DNA from the mammal and ascertaining whether a sequence of the DNA encoding (a) a protein having biological activity of myostatin, is present, and whether a sequence of the DNA encoding (b) an allelic protein lacking the activity of (a), is present. The absence of (a) and the presence of (b) indicates the presence of muscular hyperplasia in the mammal.

Of course, the mutation responsible for the lack of activity can be a naturally occurring mutation, as is the case for the Belgian Blue, Asturiana, Parthenaise or Rubia Gallega breeds, shown here.

The mammal can be a bovine, etc.

There are several methods known for determining whether a particular nucleotide sequence is present in a sample. A common method is the polymerase chain reaction. The method thus includes a step in which ascertaining whether a sequence of the DNA encoding (a) is present, and whether a sequence of the DNA encoding (b) is present includes amplifying the DNA in the presence of primers based on a nucleotide sequence encoding a protein having biological activity of myostatin.

A primer used in PCR for example, is a nucleic acid molecule sufficiently complementary to the sequence on which it is based and of sufficient length to selectively hybridize to the corresponding portion of a nucleic acid molecule intended to be amplified and to prime synthesis thereof under *in vitro* conditions commonly used in PCR. Likewise, a probe, is a molecule, for example a nucleic acid molecule of sufficient length and sufficiently complementary to the nucleic acid molecule of interest, which selectively binds under high or low stringency conditions with the nucleic acid sequence of interest for detection thereof in the presence of nucleic acid molecules having differing sequences.

Primers are based on the sequence identified as SEQ ID NO:7 (human cDNA sequence) or SEQ ID NO:54.

Further described is a method for determining the presence of muscular hyperplasia in a mammal which includes obtaining a sample of material containing mRNA from the mammal. Such method includes ascertaining whether a sequence of the mRNA encoding (A) a protein having biological activity of myostatin, is present, and whether a sequence of the mRNA encoding (B) a protein at least partially encoded by a truncated nucleotide sequence corresponding to substantially the sequence of the mRNA and lacking the activity of (A), is present The absence of (A) and the presence of (B) indicates the presence of muscular hyperplasia in the mammal.

The mRNA encoding (A) and the truncated sequence can correspond to alleles of DNA of the mammal.

Again, if an amplification method such as PCR is used in ascertaining whether a sequence of the mRNA encoding (A) is present, and whether a sequence of the mRNA encoding (B) is present, the method includes amplifying the mRNA in the presence of a pair of primers complementary to a nucleotide sequence encoding a protein having biological activity of myostatin. Each such primer can contain a nucleotide sequence substantially complementary, for example, to the sequence identified as SEQ ID NO:7. The truncated sequence can contain at least 50 consecutive nucleotides substantially corresponding to 50 consecutive nucleotides of SEQ ID NO:7, for example.

Further described is a method for determining the presence of muscular hyperplasia in a mammal which includes obtaining a tissue sample of containing mRNA of the mammal and ascertaining whether an mRNA encoding a mutant type myostatin protein lacking biological activity of myostatin is present. The presence of such an mRNA encoding a mutant type myostatin protein indicates the presence of muscular hyperplasia in the mammal.

Further described is a method for determining the presence of muscular hyperplasia in a bovine animal. The method includes obtaining a sample of material containing DNA from the animal and ascertaining whether DNA having a nucleotide sequence encoding a protein having biological activity of myostatin is present. The absence of DNA having such a nucleotide sequence indicates the presence of muscular hyperplasia in the animal. Ascertaining whether DNA having a nucleotide sequence encoding a protein having biological activity of myostatin can include amplifying the DNA in the presence of primers based on a nucleotide sequence encoding a protein having biological activity of myostatin.

In particular, the method can be carried out using a sample from an animal in which such a bovine animal not displaying muscular hyperplasia is known to have a nucleotide sequence which is capable of hybridizing with a nucleic acid molecule having the sequence identified as SEQ ID NO:1 under stringent hybridization conditions.

It is possible that ascertaining whether DNA having a nucleotide sequence encoding a protein having biological activity of myostatin is present includes amplifying the DNA in the presence of primers based on a nucleotide sequence encoding the N-terminal and the C-terminal, respectively, of the protein having biological activity of myostatin.

Primers, say first and second primers, can be based on first and second nucleotide sequences encoding spaced apart regions of the protein, wherein the regions flank a mutation known to naturally occur and which when present in both alleles of a such an animal results in muscular hyperplasia.

It can also be that DNA of such an animal not displaying muscular hyperplasia contains a nucleotide sequence which hybridizes under stringent conditions with a nucleotide sequence encoding a protein having a sequence identified as SEQ ID NO:2 and the coding sequence of DNA of a such an animal displaying muscular hyperplasia is known to contain an 11-base pair deletion beginning at base pair no. 821 of the coding sequence, and said first primer is selected to be upstream of the codon encoding glutamic acid no. 275 and the second primer is selected to be downstream of the codon encoding aspartic acid no. 274.

Also, a DNA of such an animal not displaying muscular hyperplasia might contain a nucleotide sequence which hybridizes under stringent conditions with a nucleotide sequence encoding a protein having a sequence identified as SEQ ID NO:2. The coding sequence of DNA of such an animal displaying muscular hyperplasia might be known to contain an 11-base pair deletion beginning at base pair no. 821. A primer can be selected to span the nucleotide sequence including base pair nos. 820 and 821 of the DNA sequence containing the deletion.

The animal can be of the Belgian Blue breed.

In a particular aspect, ascertaining whether DNA having a nucleotide sequence encoding a protein having biological activity of myostatin is present includes amplifying the DNA in the presence of a primer containing at least a portion of a mutation known to naturally occur and which when present in both alleles of a said animal results in muscular hyperplasia.

Further described is a method for determining the presence of muscular hyperplasia in a bovine animal which includes obtaining a sample of the animal containing mRNA and ascertaining whether an mRNA encoding a protein having biological activity of myostatin is present in the sample. The absence of said mRNA indicates the presence of muscular hyperplasia in the animal.

A sample containing mRNA can be muscle tissue, particularly, skeletal muscle tissue.

A method for determining the presence of double muscling in a bovine animal, involves obtaining a sample of material containing DNA from the animal and ascertaining whether the DNA contains the nucleotide sequence identified as SEQ ID NO:11 in which the absence of the sequence indicates double muscling in the animal.

In a particular aspect, the animal is of the Belgian Blue breed.

Further described is a method for determining the myostatin genotype of a mammal, as may be desirable to know for breeding purposes. The method includes obtaining a sample of material containing nucleic acid of the mammal, wherein the nucleic acid is uncontaminated by heterologous nucleic acid; ascertaining whether the sample contains a (i) nucleic acid molecule encoding a protein having biological activity of myostatin; and ascertaining whether the sample contains an (ii) allelic nucleic acid molecule encoding a protein lacking biological activity of myostatin. The mammal can be bovine.

Further described is a method of increasing muscle mass of a mammal having muscle cells in which myostatin is expressed, the method comprising administering to the mammal an effective amount of a nucleic acid molecule substantially complementary to at least a portion of mRNA encoding the myostatin and being of sufficient length to sufficiently reduce expression of the myostatin to increase the muscle mass. In a particular aspect, the mammal is bovine.

Further described is a method of increasing muscle mass of a mammal, including administering to the mammal an effective amount of a nucleic acid molecule having ribozyme activity and a nucleotide sequence substantially complementary to at least a portion of mRNA encoding myostatin and being of sufficient length to bind selectively thereto to sufficiently reduce expression of the myostatin so as to increase the muscle mass.

The invention includes a transgenic non-human mammal having a phenotype characterized by muscular hyperplasia, said phenotype being conferred by a transgene contained in the somatic and germ cells of the mammal, the transgene encoding a myostatin protein having a dominant negative mutation. The transgenic mammal is male and the transgene is located on the Y chromosome. The mammal can be bovine and the transgene can be located to be under the control of a promoter which normally a promoter of a myosin gene.

Another transgenic non-human mammal of the invention has a phenotype characterized by muscular hyperplasia, in which the phenotype is conferred by a transgene having a sequence antisense to that encoding a myostatin protein of the mammal. The mammal can be a male bovine and the transgene can be located on the Y chromosome. The transgene can further include a sequence which when transcribed obtains an mRNA having ribozyme activity.

A transgenic non-human mammal of the invention having a phenotype characterized by muscular hyperplasia, can have the phenotype inducible and conferred by a myostatin gene flanked by J oxP sides and a Cre transgene under the dependence of an inducible promoter.

A transgenic non-human male mammal of the invention having a phenotype characterized by muscular hyperplasia, can have the phenotype conferred by a myostatin gene flanked by J oxP sides and a Cre transgene located on the Y chromosome.

The invention includes a method for determining whether a sample of mammalian genetic material is capable of a conferring a phenotype characterized by muscular hyperplasia, comprising ascertaining whether the genetic material contains a nucleotide sequence encoding a protein having biological activity of myostatin, wherein the absence of said sequence indicates the presence of muscular hyperplasia in the animal.

Further embodiments of the invention are as described in the claims.

### Brief Description Of Drawings

In describing particular aspects of the invention, reference is made to the accompanying drawings, in which:

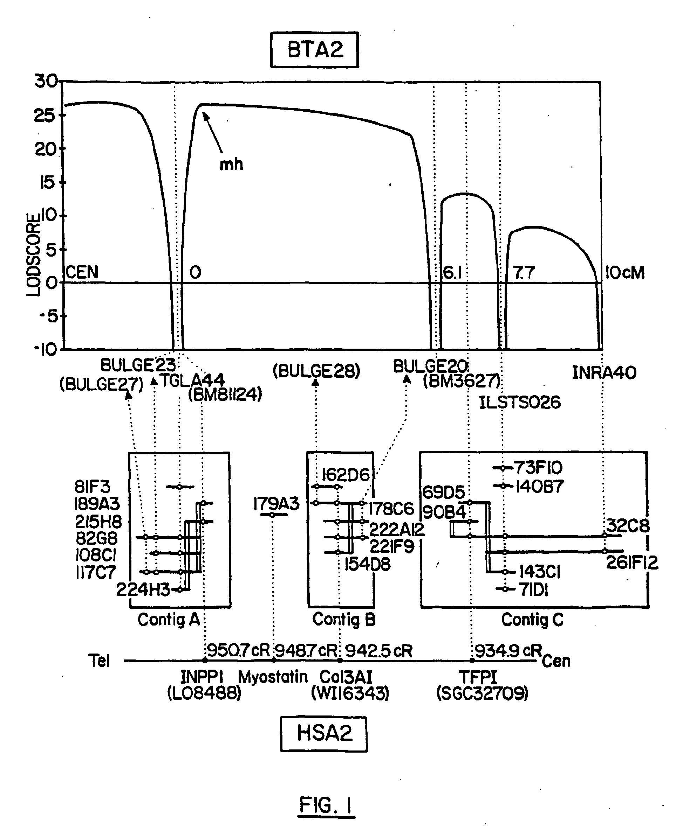

Figure 1 is a schematic summary of genetic, physical and comparative mapping information around the bovine *mh* locus. A multi-point lodscore curve obtained for the *mh* locus with respect to the microsatellite marker map is shown. Markers that were not informative in the pedigree used are shown between brackets; their map position is inferred from published mapping data. Markers and the YACs from which they were isolated are connected by arrows. The RH-map of the relevant section of human HSA2 is shown, with the relative position in cR of the ESTs used. Stippled lines connect microsatellite and Type I markers with their respective positive YACs. YACs showing cross-hybridizing SINE-PCR products are connected by the red boxes.

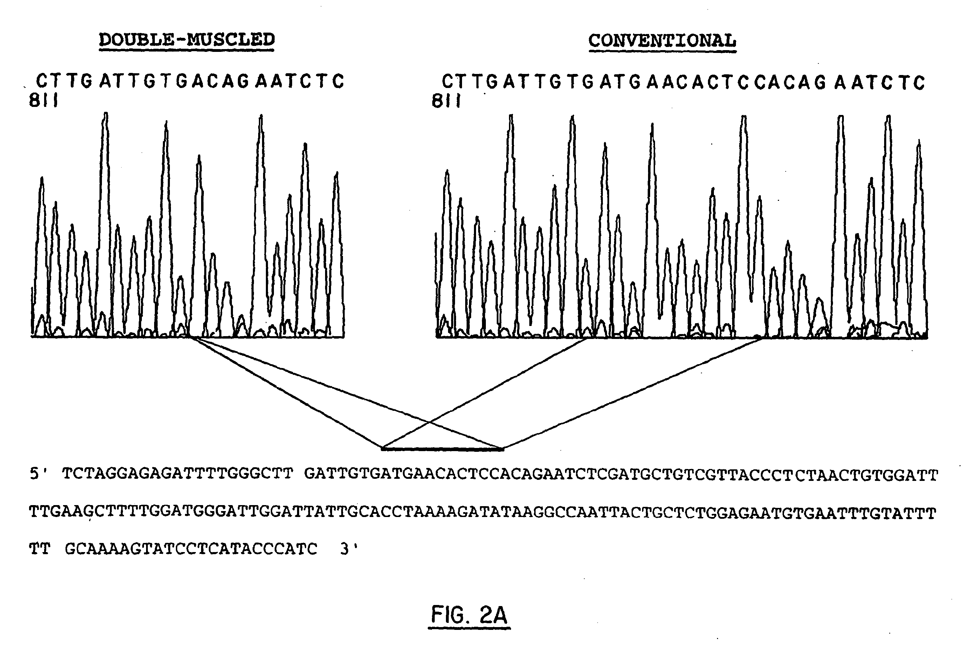

Figure 2(a) shows electropherograms obtained by cycle-sequencing the myostatin cDNA sequence from a double-muscled and a conventional animal, showing the *nt821de1(11)* deletion (SEQ ID NO:11) in the double-muscled animal. The primers used to amplify the fragment encompassing the deletion from genomic DNA are spaced apart from the remaining nucleotides.

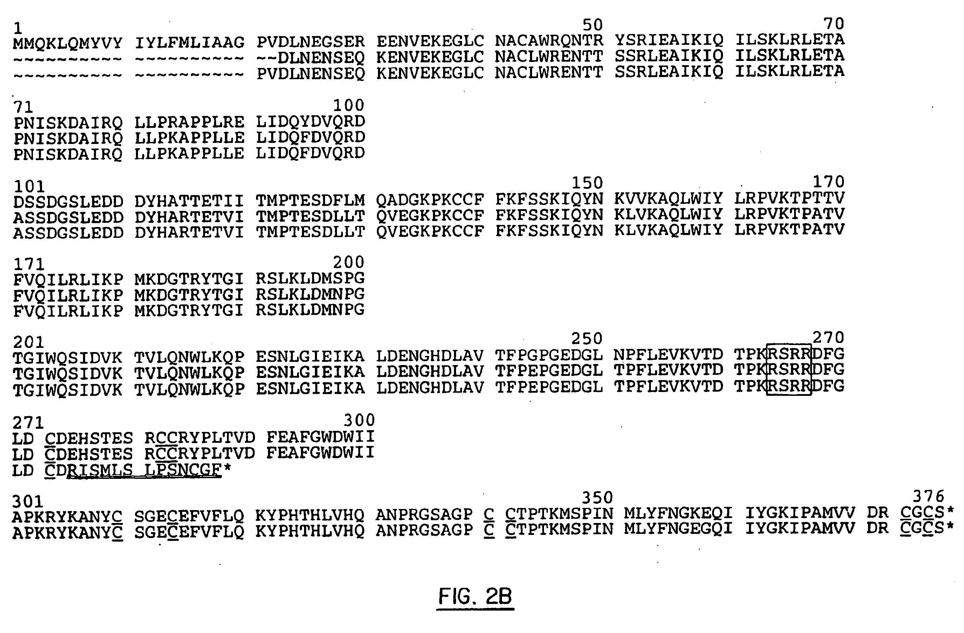

Figure 2(b) shows the amino-acid sequence of the murine (top row), bovine normal (middle row) and bovine *nt821del(11)* (bottom row) allele. The putative site of proteolytic processing is boxed, while the nine conserved cysteine in the carboxy-terminal region are underlined. The differences between the normal and *nt821del(11)* bovine allele are indicated by the double underlining.

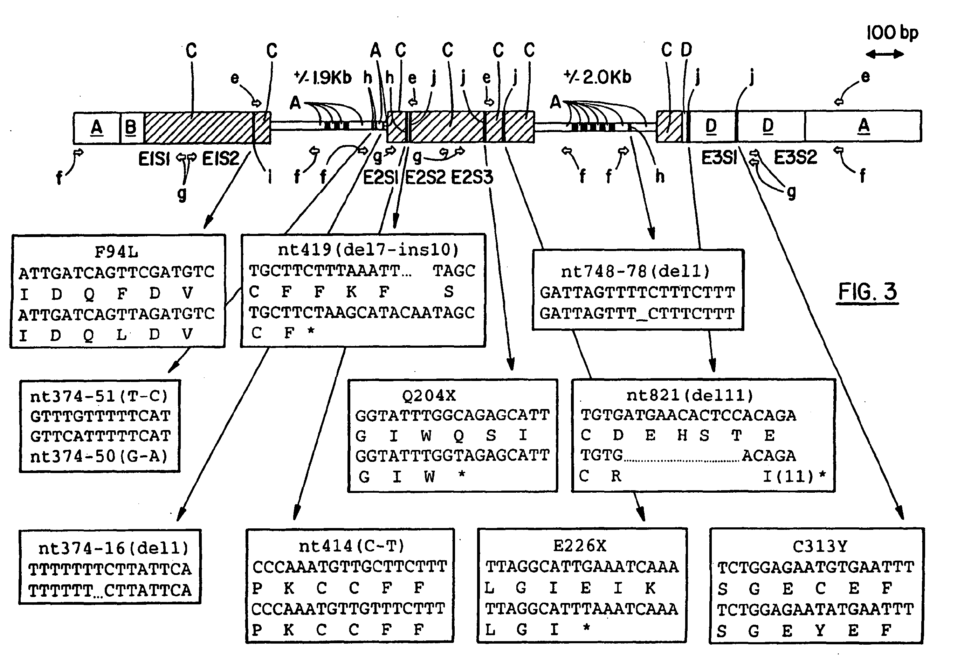

Figure 3 is a schematic representation of the bovine myostatin gene with position and definition of the identified DNA sequence polymorphisms. The and (clear) boxes correspond to the untranslated leader and trailer sequences (large diameter), and the intronic sequences (small diameter) respectively. The "B", "C", and "D" boxes correspond to the sequences coding for the leader peptide, N-terminal latency-associated peptide and bioactive carboxyterminal domain of the protein respectively. Small "e", "f" and "g" arrows point towards the positions of the primers used for intron amplification, exon amplification and sequencing and exon sequencing respectively, the corresponding primer sequences are reported in Table 1. The positions of the identified DNA sequence polymorphisms are shown as "h", "i" or "j" lines on the myostatin gene for silent, conservative and disrupting mutations respectively. Each mutation is connected via an arrow with a box reporting the details of the corresponding DNA sequence and eventually encoded peptide sequence. In each box, the valiant sequence is compared with the control Holstein-Friesian sequence and differences are highlighted in color.

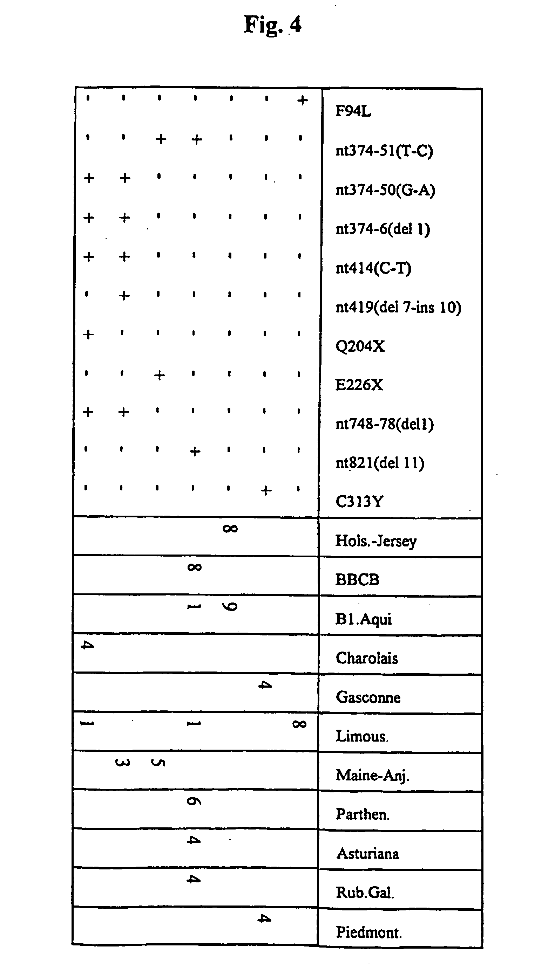

Figure 4 shows the distribution of identified mutations in the various breeds examined. The order of the myostatin mutations correspond to Figure 3. All analyzed animals were double-muscled except for the two Holstein-Friesian and two Jerseys used as controls (column 1).

### Detailed Description Of Preferred Embodiments

The method used for isolating genes which cause specific phenotypes is known as positional candidate cloning. It involves: (i) the chromosomal localization of the gene which causes the specific phenotype using genetic markers in a linkage analysis; and (ii) the identification of the gene which causes the specific phenotype amongst the "candidate" genes known to be located in the corresponding region. Most of the time these candidate genes are selected from available mapping information in humans and mice.

The tools required to perform the initial localization (step (i) above) are microsatellite marker maps, which are available for livestock species and are found in the public domain (Bishop *et al*., 1994; Barendse *et al*., 1994; Georges *et al*., 1995; and Kappes, 1997). The tools required for the positional candidate cloning, particularly the YAC libraries, (step (ii) above) are partially available from the public domain. Genomic libraries with large inserts constructed with Yeast Artificial Chromosomes ("YAC") are available in the public domain for most livestock species including cattle. When cross-referencing the human and mice map, it is necessary to identify the positional candidate, which is available at low resolution but needs to be refined in every specific instance to obtain the appropriate level of high resolution. A number of original strategies are described herein to achieve this latter objective. For general principles of positional candidate cloning, see Collins, 1995 and Georges and Andersson, 1996.

In order to allow for cross-referencing between the bovine and human gene map as part of the positional candidate cloning approach, HSA2q31-32 (map of the long arm of human chromosome 2, cytogenetic bands q31-32) and BTA2q12-22 (map of the arm of bovine chromosome 2, cytogenetic bands q12-22) were integrated on the basis of coincidence bovine YAC's as described below.

Using a previously described experimental [(normal x double-muscled) x double-muscled] backcross population comprising 108 backcross individuals, the *mh* locus was recently mapped by linkage analysis to the centromeric tip of bovine chromosome 2 (BTA2), at 3.1 centiMorgan proximal from the last marker on the linkage map: TGLA44 (Charlier *et al*., 1995). It was also known from previous work that pro-α(III) collagen *(Col3Al)* was located in the same chromosomal region as the *mh* locus. *Col3Al* has been mapped to BTA2q12-22 by *in situ* hybridization (Solinas-Toldo *et al*., 1995), while a *Col3Al* RFLP marker was shown to be closely linked to TGLA44 (θ=2%)(Fisher *et al*., 1996). This identifies the region flanking *Col3Al* on the human map, i.e. HSA2q31-32, as the likely orthologous human chromosome segment. This assumption is compatible with data from Zoo-FISH experiments (Solinas-Toldo *et al*., 1995) as well as mapping data of Type I markers on somatic cell hybrids (O'Brien *et al*., 1993), which establish an evolutionary correspondence between segments of HSA2q and BTA2.

In order to refine the correspondence between the HSA2q31-33 and BTA2q12-22 maps, Comparative Anchored Tagged Sequences or CATS, i.e. primer pairs that would amplify a Sequence Tagged Site or STS from the orthologous gene in different species (Lyons *et al*., 1996), were developed for a series of genes flanking *Col3A1* on the human map and for which sequence information was available in more than one mammal. In addition to *Col3Al*, working CATS were obtained for α2(V) collagen (*Col5A2*), inositol polyphosphate-1 phosphatase (*INPP1*), tissue factor pathway inhibitor precursor (*TFPI*), titin (*TTN*), n-chimaerin (*CHN*), glutamate decarboxylase 67 (*GAD1*), Cytotoxic T-lymphocyte-associated protein 4 (*CTLA4*) and T-cell membrane glycoprotein CD28 (*CD28*). The corresponding primer sequences are given in Table 1.

**Table 1:**

| CATS | | |
|---|---|---|
| INPP1 | UP: 5' CAGCAAAGTCCTTAATGGTAACAAGC 3' | DN: 5' GGGTCACTGAAGAAAACGTCCTG 3' |
| COL3A1 | UP: 5' CCCCATATTATGGAGATGAACCG 3' | DN: 5' AGTTCAGGATGGCAGAATTTCAG 3' |
| COL5A2 | UP: 5' GCAAACTGGGYGGRAGCAAGACC 3' | DN: 5' TTSTTCCTGGGCTTTTATTGAOAC 3' |
| TFPI | UP: 5' AAGCCWGATTTCTGCTTYTTGGAAG 3' | DN: 5' TGCCMAGGCAHCCRCCRTACTTGAA 3' |
| TTN | UP: 5' GGTCGTCCTACACCAGAAG 3' | DN: 5' GGTTGACATTGTCAAGAACAAG 3' |
| CHN | UP: 5' TCTCMAAAGTCGTCTGTGACAATC 3' | DN: 5' TGYTCRTTTTCTTTCAGAGTTGC 3' |
| GAD1 | UP: 5' RCTGGTCCTCTTCACCTCAGAAC 3' | DN: 5' ACATTGTCVGTTCCAAAGCCAAG 3' |
| CTLA4 | UP: 5' AGGTYCGGGTGACDGTGCTKC 3' | DN: 5' TGGRTACATGAGYTCCACCTTGC 3' |
| CD28 | UP: 5' AGCTGCARGTATWCCTACAAYCT 3' | DN: 5' GTYCCRTTGCTCYTCTCRTTGYC 3' |

| Microsatellite markers | | |
|---|---|---|
| TGLA44 | UP:5' AACTGTATATTGAGAGCCTACCATG 3' | DN: 5' CACACCTTAGCGACTAAACCACCA 3' |
| BULGE27 | UP: 5' CTACCTAACAGAATGATTTTGTAAG 3' | DN: 5' AGTGTTCTTGCCTAGAGAATCCCAG 3' |
| BULGE23 | UP: 5' ACATTCTCTCACCAATATGACATAC 3' | DN: 5' TAAGTCACCATTACATCCTTAGAAC 3 |
| BM81124 | UP: 5' GCTGTAAGAATCTTCATTAAGCACT 3' | DN: 5' CCTGATACATGCTAAGGTTAAAAAC 3" |
| BULGE28 | UP: 5' AGGCATACATCTGGAGAGAAACATG 3' | DN: 5' CAGAGGAGCCTAGCAGGCTACCGTC 3' |
| BULGE20 | UP: 5' CAGCAGGTCTGTTGAAGTGTATCAG 3' | DN: 5' AGTGGTAGCATTCACAGGTAGCCAG 3' |
| BM3627 | UP: 5' CAGTCCATGGCACCATAAAG 3' | DN: 5' TCCGTTAGTACTGGCTAATTGC 3' |
| ILSTS026 | UP: 5' CTGAATTGGCTCCAAAGGCC 3' | DN: 5' AAACAGAAGTCCAGGGCTGC 3' |
| INRA40 | UP: 5' TCAGTCTCCAGGAGAGAAAAC 3' | DN: 5' CTCTGCCCTGGGGATGATTG 3' |

| Bovine Myostatin primers | | |
|---|---|---|
| GDF8.19 | 5' AATGTATGTTTATATTTACCTGTTCATG 3' | |
| GDF8.11 | 5' ACAGTGTTTGTGCAAATCCTGAGAC 3' | |
| GDF8.12 | 5' CAATGCCTAAGTTGGATTCAGGTTG 3' | |
| GDF8.25 | 5' CTTGCTGTAACCTTCCCAGGACCAG 3' | |
| GDF8.15 | 5' TCCCATCCAAAGGCTTCAAAATC 3' | |
| GDF8.21 | 5' ATACTCWAGGCCTAYAGCCTGTGGT 3' | |

| | | |
|---|---|---|
| Reading from left to right and down the table, the sequences given in Table 1 are identified as SEQ ID NO:12 to SEQ ID NO:53, respectively. | | |

These CATS were used to screen a 6-genome equivalent bovine YAC library by PCR using a three-dimensional pooling strategy as described by Libert *et al*., 1993. The same YAC library was also screened with all microsatellite markers available for proximal BTA2, i.e. TGLA44, BM81124, BM3627, ILSTS026, INRA40 and TGLA431 (Kappes *et al*., 1997).

Potential overlap between the YACs obtained with this panel of STS's was explored on the basis of common STS content, as well as cross-hybridization between SINE-PCR product from individual YACs. From this analysis, three independent YAC contigs emerged in the region of interest: (i) contig A containing microsatellites TGLA44, BM81124 and Type I marker *INPP1*; (ii) contig B containing *Col3Al* and *Col5A2*; and (iii) contig C containing microsatellite markers BM3627, ILSTS026 and INRA40, and Type I marker TFPI.

None of the available microsatellites mapped to contig B, therefore this cluster of YACs could not be positioned in cattle with respect to the two other contigs. Available mapping information in the human, however, allowed prediction of contig B's position between contigs A and C. To test this hypothesis, two new microsatellite markers were isolated from contig B, BULGE20 and BULGE28. BULGE20 proved to be polymorphic, allowing for genotyping of the experimental backcross population.

In addition, to increase the informativeness of the markers available for contig A, two new microsatellite markers were developed from this contig: BULGE23 and BULGE27. BULGE23 proved to be polymorphic and was used to type the same pedigree material.

All resulting genotypes were used to construct a linkage map using the ILINK program (Lathrop and Lalouel, 1984). The following most likely order and sex-averaged recombination rates between adjacent markers was obtained: [TGLA44-(0%)-BULG23]-(6,1%)-BULG20-(1,6%)-ILSTS026-(2.3%)-INRA40-(7,1%)-TGLA431. The position of BULGE20 between TGLA44 and ILSTS026 confirmed the anticipated order of the three contigs. Figure 1 summarizes the resulting mapping information.

A multi point linkage analysis was undertaken using LINKMAP, to position the *mh* locus with respect to the new marker map. Linkage analysis was performed under a simple recessive model, assuming full penetrance for *mh*/*mh* individuals and zero penetrance for the two other genotypes. The LOD score curve shown in Figure 1 was obtained, placing the *mh* locus in the TGLA44-BULGE20 interval with an associated maximum LOD score of 26.4. Three backcross individuals were shown to recombine with the BULGE20 and distal markers, but not with TGLA44 and BULGE23, therefore placing the *mh* locus proximal from this marker. One individual, was shown to recombine with TGLA44 and BULGE23, but not with the more distal markers, therefore positioning the *mh* locus distal from TGLA44 and BULGE23. Given the relative position of these microsatellite markers with respect to *INPP1* and *Col3Al* as deduced from the integration of the human and bovine map, these results indicated that the *mh* gene is likely located in a chromosome segment bounded by *INPP1* and *Col3Al*.

Recently, McPherron *et al*. (1997) demonstrated that mice homozygous for a knock-out deletion of *GDF-8* or *myostatin*, were characterized by a generalized increase in skeletal muscle mass. Using the published 2676bp murine *myostatin* cDNA sequence (GenBank accession number U84005), a Tentative Human Consensus (THC) cluster in the Unigene database was identified which represented three cDNA clones (221299, 300367, 308202) and six EST (Expressed Sequence Tag) sequences (H92027, H92028, N80248, N95327, W07375, W24782). The corresponding THC covered 1296 bp of the human *myostatin* gene, showing an homology of 78.1% with the murine sequence when averaged over the entire sequence, and 91.1 % when considering only the translated parts of the human and murine genes (566bp). This THC therefore very likely corresponds to the human orthologue of the murine *myostatin* gene. Primers (5'-GGCCCAACTATGGATATATTTG-3' (SEQ ID NO:9) and 5'-GGTCCTGGGAAGGTTACAGCA-3' (SEQ ID NO:10)) were thus prepared to amplify a 272 bp fragment from the second exon of human *myostatin* and used to genotype the whole-genome Genebridge-4 radiation hybrid panel (Walter *et al*., 1994). The *RHMapper* program (Slonim *et al*., unpublished) was used to position the *myostatin* gene with respect to the Whitehead/MIT framework radiation hybrid map, placing it at position 948.7 cR of the HSA2 map with an associated lodscore > 3. Closer examination of the myostatin segregation vector and its confrontation with the vectors from all markers located in that region (Data Release 11.9, May 1997) showed it to be identical to EST SGC38239 placed on the Whitehead/MIT radiation hybrid map (Hudson *et al*., 1995) at position 946.8 cR of HSA2. This places the human *myostatin* gene on the RH-map in the interval between *Col3Al* (EST WI16343 - 942.5 cR) and *INPP1* (EST L08488 - 950.2 to 951.2 cR)(Figure 1). *Myostatin* therefore appeared as a very strong positional candidate for the *mh* gene.

To test the potential involvement of *myostatin* in the determinism of double-muscling in cattle, primer pairs were designed based on the available mouse and human *myostatin* sequence, with the objective to amplify the entire coding sequence from bovine cDNA using PCR (Polymerase Chain Reaction). Whenever possible, primers were therefore positioned in portions of the *myostatin* sequence showing 100% homology between mouse and human. Two primer pairs were identified that amplified what was predicted to represent 98.4% of the bovine coding sequence plus 74 bp of 3' untranslated sequence, in two overlapping DNA fragments, respectively 660 (primers GDF8.19 - GDF8.12) and 724 bp (primers GDF8.11 - GDF8.21) long. The expected DNA products were successfully amplified from cDNA generated from skeletal muscle of both a normal (homozygous +/+) (SEQ ID NO:1) and a double-muscled (homozygous *mh*/*mh*) (SEQ ID NO:3) animal, and cycle-sequenced on both strands.

The nucleotide sequence corresponding to the normal allele presented 88.9% identity with the mouse myostatin sequence (SEQ ID NO:5) over a 1067 bp overlap, and contained the expected open reading frame encoding a protein (SEQ ID NO:2) showing 92.9% identity in a 354 amino-acid overlap with mouse myostatin (SEQ ID NO:6). As expected for a member of the TGFβ superfamily, the bovine myostatin gene is characterized by a proteolytic processing site thought to mediate cleavage of the bioactive carboxy-terminal domain from the longer N-terminal fragment, and by nine cysteine residues separated by a characteristic spacing and suspected to be involved in intra- and inter-molecular disulfide bridges (McPherron and Lee, 1996).

The nucleotide sequence obtained from the *mh* allele was identical to the normal allele over its entire length, except for an 11 bp deletion involving nucleotides 821 to 831 (counting from the initiation codon). This frame shifting deletion, occurring after the first cysteine residue of the carboxy-terminal domain, drastically disrupts the downstream amino-acid sequence and reveals a premature stop-codon after 13 amino acids, see Figure 2. The amino acid sequence encoded by the mutant nucleic acid sequence is identified as SEQ ID NO:4. This mutation disrupts the bioactive part of the molecule and is therefore very likely to be the cause of the recessive double-muscling phenotype. Following conventional nomenclature, this mutation wilt be referred to as *nt821(del11)*.

To further strengthen the assumption of the causality of this mutation, primer pairs flanking the deletion (Figure 2) were prepared and the corresponding DNA segment from all animals from the experimental backcross population amplified. PCR was performed in the presence of dCTP³² in order to radioactively label the amplification product. Amplification products were separated on denaturing polyacrylamide gels and detected by autoradiography. A 188 bp product would be expected for the normal allele and a 177 bp product for the *nt821(del11)* allele. Correlation between phenotype and genotype was matched for the entire pedigree. All ten BBCB double-muscled sires were found to be homozygous for the *nt821(del11)* mutation, all 41 F1 females were heterozygous, while 53 double-muscled offspring were homozygous for the mutation, the remaining 55 conventional animals were heterozygous.

To examine the distribution of the *nt821(del11)* mutation in different conventional and double-muscled breeds, a cohort of 25 normal individuals was genotyped representing two dairy breeds (Nolstein-Friesian, Red-and -White) and a cohort of 52 double-muscled animals representing four breeds (BBCB, Asturiana, Maine-Anjou and Piémontese). The results are summarized in Table 2. All dairy animals were homozygous normal except for one Red-and-White bull shown to be heterozygous. The occurrence of a small fraction of individuals carrying the mutation in dairy cattle is not unexpected as the phenotype is occasionally described in this breed. In BBCB and Asturiana, all double-muscled animals were homozygous for the *nt821*(*dal11*) deletion, pointing towards allelic homogeneity in these two breeds. Double-muscled Maine-Anjou and Piémontese animals were homozygous "normal", i.e. they did not show the *nt821(del11)* deletion but a distinct cysteine to tyrosine substitution (*C313Y*) in double-muscled Piédmontese animals identified by others (Kambadur *et al*., 1997) was discovered.

**Table 2:**

| Breed | Phenotype | Genotype | | |
|---|---|---|---|---|
| | | +/+ | *+*/*nt821(del11)* | *nt821(del11)*/*nt821(del11)* |
| Belgian Blue | DM | | | 29 |
| Asturiana | DM | | | 10 |
| Piémontese | DM | 8 | | |
| Maine-Anjou | DM | 4 | | |
| Holstein-Friesian | Normal | 13 | | |
| Red-and-White | Normal | 12 | 1 | |

The entire coding sequence was also determined for the *myostatin* gene in double-muscled individuals from ten European cattle breeds and a series of mutations that disrupt myostatin function were identified.

The coding sequence of four control Holstein-Friesian and Jersey individuals was identical to the previously described wild-type allele (Grobet *et al*., 1997), further indicating that it was the genuine myostatin coding sequence being amplified, and not a non-functional pseudogene.

Amongst the 32 double-muscled animals, seven DNA sequence variants within the coding region were found, as summarized In Figure 3.

In addition to the *nt821(del11)* mutation in the third exon, described above, four new mutations that would be expected to disrupt the myostatin function were found. An insertion/deletion at position 419 counting from the initiation codon, replacing 7 base pairs with an apparently unrelated stretch of 10 base pairs, reveals a premature stop codon in the N-terminal latency-associated peptide at amino-acid position 140. This mutation is referred to as *nt419(del7-ins10)*. Two base pair substitutions in the second exon, a C→T transition at nucleotide position 610 and a G→T transversion at nucleotide position 676, each yield a premature stop codon in the same N-terminal latency-associated peptide at amino-acid positions 204 and 226 respectively. These mutations are called Q204Xand E226X respectively. Finally, a G→A transition at nucleotide position 938 results in the substitution of a cysteine by a tyrosine. This mutation is referred to as *C313Y*. This cysteine is the fifth of nine highly conserved cysteine residues typical of the members of the TGF-β superfamily and shared in particular by TGF-β1, - β2 and -β3, and inhibin-βA and -βB (McPherron & Lee, 1996). It is thought to be involved in an intramolecular disulfide bridge stabilizing the three-dimensional conformation of the bioactive carboxyterminal peptide. Its substitution is therefore likely to affect the structure and function of the protein. This *C313Y* has recently also been described by Kambadur *et al*. (1997).

A conservative phenylalanine to leucine substitution was also found at amino-acid position 94 in the first exon, due to a C→A transversion at nucleotide position 282 of the *myostatin* gene. Given the conservative nature of the amino-acid substitution, its location in the less conserved N-terminal latency-associated peptide, and as this mutation was observed at the homozygous condition in animals that were not showing any sign of exceptional muscular development, this mutation probably does not interfere drastically with the myostatic function of the encoded protein, if at all. This mutation is referred to as *F94L*. The murine protein is characterized by a tyrosine at the corresponding amino-acid position.

Also identified was a silent C→T transition at the third position of the 138th cytosine codon in the second exon, referred to as *nt414(C-T)*.

In addition to these DNA sequence polymorphisms detected in the coding region of the *myostatin* gene, also found were four DNA sequence variants in intronic sequences which are probably neutral polymorphisms and which have been assigned the following symbols: *nt374-51(T-C), nt374-50(G-A), nt374-16(del1)* in intron 1, and *nt748-78(del1)* in intron 2 (Figure 3).

Figure 4 shows the observed distribution of mutations in the analysed sample sorted by breed. For the majority of the studied breeds, the analyzed double-muscled animals were homozygous for one of the five described mutations expected to disrupt the myostatin function or compound heterozygotes for two of these mutations. This is compatible with the hypothesis that the double-muscled condition has a recessive mode of inheritance in all these breeds.

Only in Limousin and Blonde d'Aquitaine was there no clear evidence for the role of myostatin loss-of-function mutations in the determinism of the observed muscular hypertrophy. Most Limousin animals were homozygous for the conservative F94L substitution which is unlikely to cause the muscular hypertrophy characterizing these animals, as discussed above. One Limousin animal proved to be heterozygous for this mutation, the other allele being the "wild-type" one. All Blonde d'Aquitaine animals were homozygous "wild-type". These data indicate either that the myostatin gene is possibly not involved in the double-muscled condition characterizing these two breeds, or that there are additional myostatin mutations outside of the coding region. The double-muscling condition is often considered to be less pronounced in Limousin animals compared to other breeds.

The data indicate that some mutations, such as the *nt821del(11)* and *C313Y,* are shared by several breeds which points towards gene migration between the corresponding populations, while others seems to be confined to specific breeds. Moreover, while some breeds (the Belgian Blue breed in particular) seem to be essentially genetically homogeneous others show clear evidence for allelic heterogeneity (e.g. Maine-Anjou).

The observation of allelic heterogeneity contradicts with the classical view that a single mh mutation spread through the European continent in the beginning of the 19th century with the dissemination of the Shorthorn breed from the British Isles (Ménissier, 1982). Two of the mutations at least are shared by more than one breed, indicating some degree of gene migration but definitely not from a single origin.

In mice, and in addition to the *in vitro* generated myostatin knock-out mice (McPherron & Lee, 1997), the compact mutation could be due to a naturally occurring mutation at the *myostatin* gene. The *compact* locus has been mapped to the *D1Mit375-D1Mit21* interval on mouse chromosome 1 known to be orthologous to *HSA2q31-32* and *BTA2q12-22* (Varga *et al*., 1997).

From an applied point of view, the characterisation of a panel of mutations in the *myostatin* gene associated with double-muscling contributes to the establishment of a diagnostic screening system allowing for marker assisted selection for or against this condition in cattle.

### Example 1

### Genetic and physical mapping

Integration of the HSA2q31-32 and BTA2q12-22 maps was done by using coincident YAC's and the mh locus was positioned in the interval flanked by Col3Al and INPP1 as follows. Genetic mapping was performed using a previously described (Holstein-Friesian x Belgian Blue) x Belgian Blue experimental backcross population counting 108 informative individuals (Charlier *et al*., 1995). Microsatellite genotyping was performed according to standard procedures (Georges *et al*., 1995), using the primer sequences reported in Table 1. Linkage analyses were performed with the MLINK, ILINK and LINKMAP programs of the LINKAGE (version 5.1) and FASTLINK (2.3P version, June 1995) packages (Lathrop & Lalouel, 1984; Cottingham *et al*., 1993). The YAC library was screened by PCR using a three dimensional pooling scheme as described in Ubert *et al*., 1993. The primer pairs corresponding to the CATS used to screen the library are reported in Table 1. Cross-hybridisation between SINE-PCR products of individual YACs was performed according to Hunter *et al*. (1996), using primers reported in Lenstra *et al*. (1993). Microsatellites were isolated from YACs according to Cornelis *et al*. (1992).

### Example 2

### Mapping of the human myostatin gene on the Genebridge-4-panel

DNA from the Genebridge-4 panel (Walter *et al*., 1994) was purchased from Research Genetics (Huntsville, Alabama), and genotyped by PCR using standard procedures and the following human *myostatin* primer pair (5'-GGCCCAACTATGGATATATTTG-3' and 5'-GGTCCTGGGAAGGTTACAGCA-3'). Mapping was performed via the WWW server of the Whitehead Institute/MIT Center for Genome Research using their *RH-mapper* program (Slonim, D.; Stein, L.; Kruglyak, L.; Lander, E., unpublished) to position the markers with respect to the framework map. Segregation vectors of the query markers were compared with the vectors from all markers in the region of interest in the complete Data Release 11.9 (May 1997) to obtain a more precise position. This positions myostatin in the INPP1-Col3Al on the human map with LOD score superior to 3.

### Example 3

### RT-PCR

To clone the bovine myostatin orthologue a strategy based on RT-PCR amplification from skeletal muscle cDNA was chosen. Total RNA was extracted from skeletal muscle (*Triceps brachialis*) according to Chirgwin *et al*. (1979). RT-PCR was performed using the Gene-Amp RNA PCR Kit (Perkin-Elmer) and the primers reported in Table 1. The PCR products were purified using QiaQuick PCR Purification kit (Qiagen) and sequenced using Dye terminator Cycle Sequencing Ready Reaction (Perkin-Elmer) and an ABI373 automatic sequencer, using the primers reported in Table 2.

### Example 4

### Diagnosis of the nt821(del11) deletion

To diagnose the nt821 (del11) the following primer sequences were designed flanking the *nt821(del11)* deletion: 5'-TCTAGGAGAGATTTTGGGCTT-3' (SEQ ID NO:53) and 5-GATGGGTATGAGGATACTTTTGC-3' (SEQ ID NO:52). These primers amplify a 188 bp fragments from normal individuals and a 177bp fragment from double-muscled Individuals. Heterozygous individuals show the two amplification products. These amplification products can be detected using a variety of methods. In this example the PCR product was labelled by incorporation of dCTP³², separated on a denaturing acrylamide gel and revealed by autoradiography. Other approaches that could be used to distinguish the three different genotypes are known to those skilled in the art and would include separation in agarose gels and visualization with ethidium bromide, direct sequencing, TaqMan assays, hybridization with allele specific oligonucleotides, reverse dot-blot, RFLP analysis and several others. The specificity of the test is linked to the detected mutation and not to the primers used in the detection method. That means that other primers can easily be designed based on said bovine myostatin sequence that would fulfill the same requirements.

### Example 5

### Determination of mutations in other breeds

A total of 32 animals with extreme muscular development were sampled from ten European beef cattle breeds in which double-muscled animals are known to occur at high to moderate frequency: (i) Belgium: Belgian Blue (4), (ii) France: Blonde d'Aquitaine (5), Charolais (2), Gasconne (2), Limousin (5), Maine-Anjou (4), Parthenaise (3), (iii) Spain: Asturiana (2), Rubia Gallega (2), (iv) Italy: Piedmontese (2). The determination of the double-muscled phenotype of the sampled animals was performed visually by experienced observers. Four animals with conventional phenotype sampled from the Holstein-Friesian (2) and Jersey (2) dairy populations were analysed as controls.

In order to facilitate the study of the myostatin coding sequence from genomic DNA, the sequences of the exon-intron boundaries of the bovine gene were determined. In mice, the *myostatin* gene is known to be interrupted by two introns, respectively ≈ 1.5 and 2.4 Kb long (McPherron & Lee, 1997). Two primer pairs were thus designed, respectively, in bovine exons 1 and 2, and exons 2 and 3, that were predicted to flank the two introns, assuming conservation of gene organisation between mouse and cattle (Figure 3 and Table 3). Using these primer sets, two PCR products respectively 2Kb and 3.5Kb long were generated from a YAC clone (179A3) containing the bovine myostatin gene (Grobet *et al*., 1997). The PCR products were purified using QiaQuick PCR Purification kit (Qiagen) and partially sequenced using Dye terminator Cycle Sequencing Ready Reaction (Perkin-Elmer) and an ABI373 automatic sequencer. Alignment with the bovine cDNA sequence identified the four predicted exon-intron boundaries. The nucleotide sequence corresponding to bovine genomic DNA is identified as SEQ ID NO:54.

**Table 3: Primers used for PCR amplification and cycle sequencing.**

| | | | |
|---|---|---|---|
| Intron1-5' | 5'-GAAGACGATGACTACCAC GCCAGGACG-3' | Intron1-3' | 5'-CTAGTTTATTGTATTGTATCTT AGAGC-3' |
| Intron2-5' | 5'-AGACTCCTACAACAGTGT TTGT-3'. | Intron2-3' | 5'-ATACTCWAGGCCTAYAGCCT GTGGT-3' |
| Exon1-5' | 5'-ATTCACTGGTGTGGCAAG TTGTCTCTCAGA-3' | Exon1-3' | 5'-CCCTCCTCCTTACATACAAGC CAGCAG-3' |
| Exon2-5' | 5'-GTTCATAGATTGATATGG AGGTGTTCG-3' | Exon2-3' | 5'-ATAAGCACAGGAAACTGGTAG TTATT-3' |
| Exon3-5' | 5'-GAAATGTGACATAAGCAA AATGATTAG-3' | Exon3-3' | 5'-ATACTCWAGGCCTAYAGCCT GTGGT-3' |
| Exon1-Seq1 | 5'-TTGAGGATGTAGTGTTTT CC-3' | Exon1-Seq2 | 5'-GCCATAAAAATCCAAATCCTC AG-3' |
| Exon2-Seq1 | 5'-CATTTATAGCTGATCTTC TAACGCAAG-3' | Exon2-Seq2 | 5'-TGTCGCAGGAGTCTTGACAG GCCTCAG-3' |
| Exon2-Seq3 | 5'-GTACAAGGTATACTGGAA TCCGATCTC-3' | | |
| Exon3-Seq1 | 5'-AGCAGGGCCGGCTGAA CCTCTGGG-3' | Exon3-Seq2 | 5'-CCCCAGAGGTTCAGCCGGCC CCTGC-3' |

Based on the available exonic and intronic sequences of the bovine myostatin gene, three primer pairs that jointly allow for convenient amplification of the entire coding sequence from genomic DNA were designed. The position of the corresponding primers is shown in Figure 3, and the corresponding sequences are reported in Table 3.

After PCR amplification of the entire coding sequence from genomic DNA in the three described fragments, these were purified using QiaQuick PCR Purification kit (Qiagen) and sequenced using Dye terminator Cycle Sequencing Ready Reaction (Perkin-Elmer) and an ABI373 automatic sequencer, using the primers used for amplification as well as a series of nested primers (Figure 3 and Table 3). Chromat files produced with the ABI373 sequencer were analysed with the *Polyphred* application (D. Nickerson, personal communication), which is part of a series of sequence analysis programs including *Phred* (Ewing, B. & Green, P. (1992), unpublished), *Phrap* (Green, P. (1994), unpublished) and Consed (Gordon, D. (1995), unpublished), but any suitable sequencing programme would do, as known to a person skilled in the art.

Antisense nucleic acids or oligonucleotides (RNA or preferably DNA) can be used to inhibit myostatin production in order to increase muscle mass of an animal. Antisense oligonucleotides, typically 15 to 20 bases long, bind to the sense mRNA or pre mRNA region coding for the protein of interest, which can inhibit translation of the bound mRNA to protein. The cDNA sequence encoding myostatin can thus be used to design a series of oligonucleotides which together span a large portion, or even the entire cDNA sequence. These oligonucleotides can be tested to determine which provides the greatest inhibitory effect on the expression of the protein (Stewart, 1996). The most suitable mRNA target sites include 5'- and 3'-untranslated regions as well as the initiation codon. Other regions might be found to be more or less effective Alternatively, an antisense nucleic acid or oligonucleotide may bind to myostatin coding or regulatory sequences.

Nucleic acids which encode myostatin proteins can be used to generate transgenic animals. A transgenic animal (e.g., a mouse) is an animal having cells that contain a transgene, which transgene is introduced into the animal or an ancestor of the animal at a prenatal, e.g., an embryonic stage. A transgene is a DNA which is integrated into the genome of a cell from which a transgenic animal develops. In one embodiment, a bovine cDNA, comprising the nucleotide sequence shown in SEQ ID NO:1, or an appropriate variant or subsequence thereof, can be used to generate transgenic animals that contain cells which express bovine myostatin. Likewise, variants such as mutant genes (e.g. SEQ ID NO:3) can be used to generate transgenic animals. This could equally well be done with the human myostatin protein and variants thereof. "Knock out" animals, as described above, can also be generated. Methods for generating transgenic animals, particularly animals such as mice, have become conventional in the art are described, for example, in U.S. Patent Nos. 4,736,866 and 4,870,009. In a preferred embodiment, plasmids containing recombinant molecules of the invention are microinjected into mouse embryos. In particular, the plasmids are microinjected into the male pronuclei of fertilized one-cell mouse eggs; the injected eggs are transferred to pseudo-pregnant foster females; and, the eggs in the foster females are allowed to develop to term. (Hogan, 1986). Alternatively, an embryonal stem cell line can be transected with an expression vector comprising nucleic acid encoding a myostatin protein, and cells containing the nucleic acid can be used to form aggregation chimeras with embryos from a suitable recipient mouse strain. The chimeric embryos can then be implanted into a suitable pseudopregnant female mouse of the appropriate strain and the embryo brought to term. Progeny harboring the transfected DNA in their germ cells can be used to breed uniformly transgenic mice.

Such animals could be used to determine whether a sequence related to an intact myostatin gene retains biological activity of myostatin. Thus, for example, mice in which the murine myostatin gene has been knocked out and containing the nucleic acid sequence identified as SEQ ID NO:1 could be generated along with animals containing the nucleic acid sequence identified as SEQ ID NO:3. The animals could be examined for display of muscular hyperplasia, especially in comparison with knockout mice, which are known to display such. In this way it can be shown that the protein encoded by SEQ ID NO:3 lacks myostatin activity within the context of this invention while the protein encoded by the nucleic acid sequence identified as SEQ ID NO:1 possesses biological activity of myostatin.

In such experiments, muscle cells would be particularly targeted for myostatin (and variants) transgene incorporation by use of tissue specific enhancers operatively linked to the encoding gene. For example, promoters and/or enhancers which direct expression of a gene to which they are operatively linked preferentially in muscle cells can be used to create a transgenic animal which expresses a myostatin protein preferentially in muscle tissue. Transgenic animals that include a copy of a myostatin transgene introduced into the germ line of the animal at an embryonic stage can also be used to examine the effect of increased myostatin expression in various tissues.

The transgenic animals of the invention can be used to investigate the molecular basis of myostatin action. For example, it is expected that myostatin mutants in which one or more of the conserved cysteine residues has been deleted would have diminished activity in relation to a wild type myostatin protein in which all such residues are retained. Further, deletion of proteolytic cleavage site would likely result in a mutant lacking biological activity of myostatin.

Transgenesis can be used to inactivate myostatin activity. This could be achieved using either conventional transgenesis, i.e. by injection in fertilized oocytes, or by gene targeting methods using totipotent cell lines such as ES (embryonic stem cells) which can then be injected in oocytes and participate in the development of the resulting organisms or whose nucleus can be transferred into unfertilized oocytes, nucleus transfer or cloning.

It is also possible to create a genetically altered animal in which the double-muscling trait is dominant so that the animal would be more useful in cross-breeding. Further, in a particular aspect, the dominant trait would be male specific. In this way, bulls would be double-muscled but cows would not be. In addition, or alternatively, the trait would also be unexpressed until after birth or inducible. If inducible the trait could be induced after birth to avoid the calving difficulties described above.

There are at least three approaches that can be taken to create a dominant "*mh*" allele. Because functional myostatin, a member of the TGF-β superfamily, is a dimer, dominant negative myostatin mutations can be created (Herskowitz *et al*., 1987; Lopez *et al*., 1992). According to one method, this is accomplished by mutating the proteolytic processing site of myostatin. To enhance the dominant negative effect, the gene can be put under the control of a stronger promoter such as the CMV promoter or that of a myosin gene, which is tissue specific, i.e., expressed only in skeletal muscle. Alternatively, an antisense sequence of that encoding myostatin could be incorporated into the DNA, so that complementary mRNA molecules are generated, as understood by a person skilled in the art. Optionally, a ribozyme could be added to enhance mRNA breakdown. In another approach, are recombinase generate/ribozyme approach or the Cre-Iox P system could be used (Hoess *et al*., 1982; Gu *et al*., 1994).

Male specificity can be achieved by placing the dominant *mh* alleles on the Y chromosome by homologous recombination.

Inducibility can be achieved by choosing promoters with post-natal expression in skeletal muscle or using inducible systems such at he Tet-On and Tet-Off systems could be used (Gossen *et al*., 1992; Shockett *et al*., 1996).

Using conventional transgenesis a gene coding for a myostatin antisense is injected, for example, by inverting the orientation of the myostain gene in front of its natural promoter and enhancer sequences. This is followed by injection of a gene coding for an anti-myostain ribozyme, i.e. an RNA that would specifically bind to endogenous myostain mRNA and destroy it via its "ribozyme" activity.

Also, through gene targeting, a conventional knock-out animal can be generated, specific mutations by gene replacement can be engineered. It is possible to inactivate the myostain gene at a specific developmental time, such as after birth to avoid calving difficulties. As mentioned above, this could be achieved using the Cre-Iox P systems in which 1.ox P sides are engineered around the myostain gene by homologous recombination (gene targeting), and mating these animals with transgenic animals having a Cre transgene (coding for the Cre recombinase existing DNA flanked by J oxP sides) under the dependence of a skeletal muscle specific promoter only active after birth. This is done to obtain individuals that would inactivate their myostain gene after birth. As mentioned above, there are also gene targeting systems that allow genes to be turned on and off by feeding an animal with, for example, an antibiotic. In such an instance, one engineers an operator between the promoter of the gene and the gene itself. This operator is the target of a repressor which when binding inactivates the gene (for example, the lac operon in *E. coli*). The repressor is brought into the cell using conventional transgenesis, for example, by injection of the gene coding for the repressor.

Transgenic animals of the invention can also be used to test substances for the ability to prevent, slow or enhance myostatin action. A transgenic animal can be treated with the substance in parallel with an untreated control transgenic animal.

The antisense nucleic acids and oligonucleotides of the invention are useful for inhibiting expression of nucleic acids (e.g. mRNAs) encoding proteins having myostatin activity.

### REFERENCES

Particulars of references cited above are given below. All of the listed references are incorporated herein by reference.
Barendse, W., S.M. Armitage, L.M. Kossarek, A. Shalom, B.W. Kirkpatrick, A.M. Ryan, D. Clayton, L. U, H.L. Neibergs, N. Zhang, W.M. Grosse, J. Weiss, P. Creighton, F. McCarthy, M. Ron, A.J. Teale, R. Fries, RA. McGraw, S.S. Moore, M. Georges, M. Soller, J.E. Womack, and D.J.S. Hetzel. 1994. A genetic linkage map of the bovine genome. Nature Genet. 6: 227-235
Bishop, M.D., S.M. Kappes, J.W. Keele, R.T. Stone, S.L.F. Sunden, G.A. Hawkins, S. Solinas Toldo, R. Fries, M.D. Grosz, J. Yoo, and C.W. Beattie. 1994. A genetic linkage map for cattle. Genetics 136: 619-639.
Boss *et al*., United States Patent No. 4,816,397.
Cabilly *et al*. United States Patent No. 4,816,567.
Charlier, C.; Coppieters, W.; Farnir, F.; Grobet, L.; Leroy, P.; Michaux, C., Mni, M.; Schwers, A.; Vanmanshoven, P.; Hanset, R. & Georges, M. (1995) The mh gene causing double-muscling in cattle maps to bovine chromosome 2. Mammalian Genome 6: 788-792.
Chirgwin, J.M.; Przybyla, A.E.; MacDonald, R.J.; Rutter, W.J. (1979) Isolation of biologically active ribonucleic acid from sources enriched in ribonuclease. Biochemistry 18: 5294-5299.
Cockett, N.E.; Jackson, S.P.; Shay, T.D.; Nielsen, D.; Green, R.D.; Georges, M. (1994). Chromosomal localization of the callipyge gene in sheep (Ovis aries) using bovine DNA markers. Proceedings of the National Academy of Sciences, US, 91: 3019-3023.
Cockett, N.E.; Jackson, S.P.; Shay, T.D.; Famir, F.; Berghmans, S.; Snowder, G.; Nielsen, D.; Georges, M. (1996). Polar overdominance at the ovine callipyge locus. Science 273: 236-238.
Cole et al. (1985). Monoclonal Antibodies in Cancer Therapy. Allen R. Bliss, Inc.
Collins, F.S. 1995. Positional cloning moves from perditional to traditional. Nature Genet. 9: 347-350.
Cornelis, F.; Hashimoto, L.; Loveridge, J.; MacCarthy, A.; Buckle, V.; Julier, C.; Bell, J. (1992). Identification of a CA repeat at the TCRA locus using YACs: a general method for generating highly polymorphic markers at chosen loci. Genomics 13: 820-825.
Cottingham, R.W.; Idury, R.M.; Schäffer, A.A. (1993). Faster sequential genetic linkage computations. Am. J. Hum. Genet. 53: 252-263.
Culley, G. (1807). Observations on livestock. 4th ed., (London, G. Woodfall).
Fisher, S.R.; Beever, J.E.; Lewin, HA. (1996). Genetic mapping of COL3Al to bovine chromosome 2. Mammalian Genome 8: 76-77.
Fuji, J.; Otsu, K.; Zorzato, F.; Deleon, S.; Khanna, V.K.; Weiler, J.E. O'Brien, P.J.; MacLennan, D.H. (1991). Identification of a mutation in the porcine ryanodine receptor associated with malignant hyperthermia. Science 253: 448-451.
Georges, M.; Andersson, L (1996). Livestock genomics comes of age. Genome Research 6: 907-921.
Georges, M.; Nielsen, D.; Mackinnon, M.; Mishra, A.; Okimoto, R.; Pasquino, A.T.; Sargeant, L.S.; Sorensen, A.; Steele, M.R.; Zhao, X.; Womack, J.E.; Hoeschele, I. (1995). Mapping quantitative trait loci controlling milk production by exploiting progeny testing. Genetics 139: 907-920.
Gossen, M. & Bujard, H. (1992). Tight control of gene expression in mammalian cells by tetracycline-responsive promotors. Proceedings of the National Academy of Sciences, USA, 89: 5547-5551.
Grobet, L.; Royo Martin, L.J.; Poncelet, D.; Pirottin, D.; Brouwers, B.; Riquet, J.; Schoeberlein, A.; Dunner, S.; Ménissier, F.; Massabanda, J.; Fries, R.; Hanset, R.; Georges, M. (1997) A deletion in the myostatin gene causes double-muscling in cattle. Nature Genetics 17: 71-74.
Gu, H.; Marth, J.D.; Orban, P.C.; Mossmann, H.; Rajewsky, K. (1994). Deletion of a DNA polymerase beta gene segment in T cells using cell type-specific gene targetting. Science 265: 103-106.
Hanset, R. and Michaux, C. (1985a). On the genetic determinism of muscular hypertrophy in the Belgian White and Blue cattle breed. I. Experimental data. Génét. Sél. Evol. 17: 359-368.
Hanset, R. and Michaux, C. (1985b). On the genetic determinism of muscular hypertrophy in the Belgian White and Blue cattle breed. II. Population data. Génét. Sél. Evol. 17: 369-386.
Hanset, R. (1991). The major gene of muscular hypetrophy in the belgian Blue Cattle Breed. In Breeding for Disease Resistance in Farm Animals, Owen, Axford, eds. CA.B. International, pp.467-478.
Herskowitz, I. (1987). Functional inactivation of genes by dominant negative mutations. Nature 329:219-222. Hogan, B. et al., (1986). A Laboratory Manual, Cold Spring Harbor, N.Y., Cold Spring Harbor Laboratory.
Hoess, R.H.; Ziese, M.; Stemberg, N. (1982). P1 site-specific recombination: nucleotide sequence of the recombining sites. Proc.Natl.Acad.Sci.USA 79: 3398-3402.
Houbenwcyl, (1987). Methods of Organic Chemistry, ed. E. Wansch. Vol. 15 I and II. Thieme, Stuttgart.
Hudson et al. (1995) Science 270:1945-1954 with supplementary data from the Whitehead Institute/MIT Center for Genome Research, Human Genetic Mapping Project, data release 11.9 (May 1997)
Hunter, K; Riba, L.; Schalkwyk, L.; Clark, M.; Resenchuk, S.; Beeghly, A.; Su, J.; Tinkov, F.; Lee, P.; Ramu, E.; Lehrach, H. and Housman, D. (1996). Toward the Construction of Integrated Physical and Genetic Maps of the Mouse Genome Using Interspersed Repetitive Sequence PCR (IRS\NPCR) Genomics. Genome Research 6: 290-299.
Huse et al., (1989). Science 246: 1275-1281.
Kambadur, R.; Sharma, M.; Smith, T.P.L.; Bass, J.J. (1997). Mutations in myostatin (GDF8) in double-muscled Belgian Blue Cattle. Genome Research 7: 910-916.
Kappes, S.M.; Keele, J.W.; Stone, R.T.; McGraw, RA.; Sonstegard, T.S.; Smith, T.P.L.; Lopez-Corrales, N.L. and Beattie, C.W. (1997). A Second-Generation Linkage Map of the Bovine Genome. Genome Research 7: 235-249.
Kennett, R. (1979). Cell fusion. Methods Enzymol. 58: 345-359.
Kohler and Milstein. (1975). Nature 256: 495-497.
Kozbor et al. (1983). Immunol. Today 4: 72.
Lathrop, M.; Lalouel, J.M. (1984). Easy calculations of lodscores and genetic risk on small computers. American Journal of Human Genetics 36: 460-465.
Lenstra, J.A.; van Boxtel, J.A.F.; Zwaagstra, K.A.; Schwerin, M. (1993). Short interspersed nuclear element (SINE) sequences of the Bovidae. Animal Genetics 24: 33-39.
Libert, F.; Lefort, A.; Okimoto, R.; Georges, M. (1993) Construction of a bovine genomic library of large yeast artificial chromosome clones. Genomics 18: 270-276.
Lopez, A.R.; Cook, J.; Deininger, P.L.; Derynck, R. (1992). Dominant negative mutants of trnasforming growth factor-betal inhibit the secretation of different transforming growth factor beta isoforms. Molecular and Cellular biology 12(4):1674-1679.
Lyons, A.L.; Laughlin, T.F.; Copeland, N.G.; Jenkins, N.A.; Womack, J.E.; O'Brien, S.J. (1996). Comparative Anchor tagged Sequences for Integrative mapping of Mammalian Genomes. Nature Genetics 15: 47-56.
McPherron, A.C.; Lee, S.-J. (1996). The transforming growth factor p superfamily. In Growth Factors and Cytokines in Health and Disease, Volume 1B, pages 357-393. JAI press Inc.
McPherron, A.C.; Lawler, A.M.; Lee, S.-J. (1997). Regulation of skeletal muscle mass in mice by a new TGFβ superfamily member. Nature 387: 83-90.
Ménissier, F. (1982). Present state of knowledge about the genetic determination of muscular hypertrophy or the double muscled trait in cattle. in Current Topics in Veterinary Medicine and Animal Science, vol. 16: Muscle hypertrophy of genetic origin and its use to improve beef production, pp. 387-428. Ed. King and Ménissier, Martinus Nijhoff.
Merrifield, (1964]. J. Am. Chem. Assoc. 85: 2149-2154.
McCafferty et al., (1990). Nature 348: 552-554.
Mirski, S. and Cole, S. P. C. (1989). Antigens associated with multidrug resistance in H69AR, a small cell lung cancer cell line. Cancer Res. 49: 5719-5724.
Morrison et al., (1985). Proceedings of the National Academy of Sciences, USA, 81: 6851.
O'Brien, S.J.; Womack, J.E.; Lyons, L.A.; Moore, K.J.; Jenkins, N.A.; Copeland, N.G. (1993). Anchored reference loci for comparative genome mapping in mammals. Nature Genetics 3: 103-112.
Old, R.W. and Primrose, S.B., In: Principles of Gene Manipulation. An Introduction to Genetic Engineering, 4th ed. Oxford University Press. 63-66.
Pell, J.M.; Flint, D.J.; (1997). In: Milk Composition, Production and Biotechnology, Ed. Welch et al., Chapter 19.
Sambrook, J., Fritsch E.F. and Maniatis, T. (1989). Molecular Cloning: A Laboratory Manual. Cold Spring Harbor Lab Press, Cold Spring Harbor, New York.
Shockett, P.E.; Schatz, D.G. (1996). Diverse strategies for tetracycline-regulated inducible gene expression. Proceedings of the National Academy of Sciences, USA, 93: 5173-5176.
Solinas-Toldo, S.; Lengauer, C; Fries, R. (1995). Comparative genome map of man and cattle. Genomics 27: 489-496.
Staerz & Bevan (1986a). Proceedings of the National Academy of Sciences, USA, 83: 1453.
Staerz & Bevan (1986b). Immunology Today 7: 241.
Stewart, A.J., Canitrot, Y., Baracchini, E., Dean, N.M., Deeley, R.G., and Cole, S.P.C. (1996). Reduction of Expression of the multidrug resistance protein (MRP) in human tumor cells by antisense phophorothioate oligonucleotides. Biochem. Pharamcol. 51: 461-469.
Takeda et al., (1985). Nature 314: 452.
Tanaguchl *et al*., European Patent Publication EP171496.
Teng, et al.. (1982) Meth. Enzymol. 92: 3-16.
Varga, L.; Szabo, G.; Darvasi, A.; Müller, G.; Sass, M.; Soller, M. (1997). Inheritance and mapping of compact (Cmpt), a new mutation causing hypermuscularity in mice. Genetics, in the press.
Walter, M.A.; Spillett, D.J.; Thomas, P.; Weissenbach, J.; Goodfellow, P.N. (1994). A method for constructing radiation hybrid maps of whole genomes. Nature Genetics 7:22-28. Ward et al., (1989). Nature 341: 544-546.

### SEQUENCE ID NO. 1

1 AGGAAGAATA AGAACAAGGG AAAAGATTGT ATTGATTTTA AAACCATGCA
51 AAAACTGCAA ATCTCTGTTT ATATTTACCT ATTTATGCTG ATTGTTGCTG
101 GCCCAGTGGA TCTGAATGAG AACAGCGAGC AGAAGGAAAA TGTGCAAAAA
151 GAGGGGCTCT GTAATGCATG TTTGTGCAGG GAAAACACTA CATCCTCAAG
201 ACTAGAAGCC ATAAAAATCC AAATCCTCAG TAAACTTCGC CTGGAAACAG
251 CTCCTAACAT CAGCAAAGAT GCTATCAGAC AACTTTTGCC CAAGGCTCCT
301 CCACTCCTGG AACTGATTGA TCAGTTCGAT GTCCAGAGAG ATCCCAGCAG
351 TGACGGCTCC TTGGAAGACG ATGACTACCA CGCCAGCACG GAAACCGTCA
401 TTACCATGCC CACGGAGTCT GATCTTCTAA CGCAAGTGGA AGCAAAACCC
451 AAATGTTGCT TCTTTAAATT TAGCTCTAAG ATACAATACA ATAAACTAGT
501 AAAGGCCCAA CTGTGGATAT ATCTGAGGCC TGTCAAGACT CCTGCGACAG
551 TGTTTGTGCA AATCCTGAGA CTCATCAAAC CCATGAAAGA CGGTACAAGG
601 TATACTGGAA TCCGATCTCT GAAACTTGAC ATGAACCCAG GCACTGGTAT
651 TTGCCAGAGC ATTGATGTGA AGACACTGTT GCAGAACTGG CTCAAACAAC
701 CTGAATCCAA CTTAGGCATT GAAATCAAAG CTTTAGATCA GAATGGCCAT
751 GATCTTGCTG TAACCTTCCC AGAACCAGGA GAAGATGGAC TGACTCCTTT
801 TTTAGAAGTC AAGGTAACAG ACACACCAAA AAGATCTAGG AGAGATTTTG
851 GGCTTGATTG TGATGAACAC TCCACAGAAT CTCGATGCTG TCGTTACCCT
901 CTAACTGTGG ATTTTGAAGC TTTTGGATGG GATTGGATTA TTGCACCTAA
951 AAGATATAAG GCCAATTACT GCTCTGGAGA ATGTGAATTT GTATTTTTGC
1001 AAAAGTATCC TCATACCCAT CTTGTGCACC AAGCAAACCC CAGAGCTTCA
1051 GCCGGCCCCT GCTGTACTCC TACAAAGATG TCTCCAATTA ATATGCTATA
1101 TTTTAATGGC CAAGGACAAA TAATATACGG GAAGATTCCA GCCATGGTAG
1151 TAGATCGCTG TGGGTGTTCA TGAGTCTATA TTtGGgTTCA TAAGC

### SEQUENCE ID NO. 2

1 MQKLQISVY IYLFMLIVAG PVDLNENSEQ KENVEKEGLC NACLWRENTT

51 SSRLEAIKIQ ILSKLRLETA PNISKDAIRQ LLPKAPPLLE LIDQFDVQRD

101 ASSDGSLEDD DYHARTETVI TMPTESDLLT QVEGKPKCCF FKFSSKIQYN

151 KLVKAQLWIY LRPVKTPATV FVQILRLIKP MKDGTRYTGI RSLKLDMNPG

201 TGIWQSIDVK TVLQNWLKQP ESNLGIEIKA LDENGHDLAV TFPEPGEDGL

251 TPFLEVKVTD TPKRSRRDFG LDCDEHSTES RCCRYPLTVD FEAFGWDWII

301 APKRYKANYC SGECEFVFLQ KYPHTHLVHQ ANPRGSAGPC CTPTKMSPIN

351 MLYFNGEGQI IYGKIPAMVV DRCGCS*

### SEQUENCE ID NO. 3

1 AGGAAGAATA AGAACAAGGG AAAAGATTGT ATTGATTTTA AAACCATGCA

51 AAAACTCCAA ATCTCTGTTT ATATTTACCT ATTTATGCTG ATTGTTGCTG

101 GCCCAGTGCA TCTGAATGAG AACAGCGAGC AGAAGGAAAA TGTGGAAAAA

151 CACGGGCTGT GTAATGCATG TTTGTGGAGG GAAAACACTA CATCCTCAAG

201 ACTAGAAGCC ATAAAAATCC AAATCCTCAG TAAACTTCGC CTGGAAACAG

251 CTCCTAACAT CAGCAAAGAT GCTATCAGAC AACTTTTCCC CAAGGCTCCT

301 CCACTCCTGG AACTGATTGA TCAGTTCGAT GTCCAGAGAC ATGCCAGCAG

351 TGACGGCTCC TTGGAAGACG ATGACTACCA CGCCAGGACG CAAACGGTCA

401 TTACCATGCC CACGGAGTCT GATCTTCTAA CGCAAGTGGA AGGAAAACCC

451 AAATGTTGCT TCTTTAAATT TAGCTCTAAG ATACAATACA ATAAACTAGT

501 AAAGCCCCAA CTGTGGATAT ATCTGAGGCC TGTCAAGACT CCTGCGACAG

551 TGTTTGTGCA AATCCTCACA CTCATCAAAC CCATGAAAGA CGGTACAAGC

601 TATACTGGAA TCCGATCTCT GAAACTTGAC ATGAACCCAG GCACTGGTAT

651 TTGGCAGAGC ATTGATGTGA AGACAGTGTT GCAGAACTGG CTCAAACAAC

701 CTGAATCCAA CTTAGGCATT GAAATCAAAG CTTTAGATGA GAATGGCCAT

751 GATCTTGCTG TAACCTTCCC AGAACCAGGA GAAGATGGAC TGACTCCTTT

801 TTTAGAAGTC AAGGTAACAG ACACACCAAA AAGATCTAGG AGAGATTTTG

851 GGCTTGATTG TGACAGAATC TCGATGCTGT CGTTACCCTC TAACTGTGGA

901 TTTTGAAGCT TTTGGATGGG ATTGGATTAT TCCACCTAAA AGATATAAGG

951 CCAATTACTG CTCTGGAGAA TGTGAATTTG TATTTTTGCA AAAGTATCCT

1001 CATACCCATC TTGTGCACCA AGCAAACCCC AGAGGTTCAG CCGGCCCCTG

1051 CTGTACTCCT ACAAAGATGT CTCCAATTAA TATGCTATAT TTTAATGGCG

1101 AAGGACAAAT AATATACGGG AAGATTCCAG CCATGGTAGT AAATCGCTGT

1151 GGGTGTTCAT GACGTCTATA TTTGGTTCAT AGCTTCCTCA AACATGGAAG

1201 GTCTTCCCCT CAACAATTTT GAAACTGTTG AAATTATGT

### SEQUENCE ID NO. 4

1 MQKLQISVY IYLFMLIVAG PVDLNENSEQ KENVEKEGLC NACLWRENTT

51 SSRLEAIKIQ ILSKLRLETA PNISKDAIRQ LLPKAPPLLE LIL[?]FDVQRD

101 ASSDGSLEDD DYHARTETVI TMPTESDLLT QVEGKPKCCF FKFSSKIQYN

151 KLVKAQLWIY LRPVKTPATV FVQILRLIKP MKDGTRYTGI RSLKLDMNPG

201 TGIWQSIDVK TVLQNWLKQP ESNLGIEIKA LDENGHDLAV TFPEPGEDGL

251 TPFLEVKVTD TPKRSRRDFG LDCDRISMLS LPSNCGF

301

351

### SEQUENCE ID NO. 5

1 GTCTCTCGGA CGGTACATGC ACTAATATTT CACTTGGCAT TACTCAAAAG
51 CAAAAAGAAG AAATAAGAAC AAGCGAAAAA AAAAGATTGT GCTGATTTTT
101 AAAATGATGC AAAAACTGCA AATGTATGTT TATATTTACC TGTTCATGCT
151 GATTGCTGCT GGCCCAGTGG ATCTAAATGA GGGCAGTGAG AGAGAAGAAA
201 ATGTGGAAAA AGAGGGGCTG TGTAATGCAT GTGCGTGGAG ACAAAACACG
251 AGGTACTCCA GAATAGAACC CATAAAAATT CAAATCCTCA GTAAGCTGCG
301 CCTGGAAACA GCTCCTAACA TCAGCAAAGA TGCTATAAGA CAACTTCTGC
351 CAAGACCGCC TCCACTCCGG GAACTGATCG ATCAGTACGA CGTCCACAGG
401 GATGACACCA GTGATGGCTC TTTGGAAGAT GACGATTATC ACGCTACCAC
451 GGAAACAATC ATTACCATGC CTACAGAGTC TGACTTTCTA ATGCAAGCGG
501 ATGGCAAGCC CAAATGTTGC TTTTTTAAAT TTAGCTCTAA AATACAGTAC
551 AACAAAGTAG TAAAAGCCCA ACTGTGGATA TATCTCAGAC CCGTCAAGAC
601 TCCTACAACA GTGTTTCTGC AAATCCTGAG ACTCATCAAA CCCATGAAAG
651 ACGGTACAAG GTATACTGGA ATCCGATCTC TGAAACTTGA CATGAGCCCA
701 GGCACTGGTA TTTGGCAGAG TATTGATGTG AAGACAGTGT TGCAAAATTG
751 GCTCAAACAG CCTGAATCCA ACTTAGGCAT TGAAATCAAA GCTTTGGATG
801 AGAATGGCCA TGATCTTGCT GTAACCTTCC CAGGACCAGG AGAAGATGGG
851 CTGAATCCCT TTTTAGAACT CAAGGTGACA GACACACCCA AGAGGTCCCG
901 GAGAGACTTT GGGCTTGACT GCGATGAGCA CTCCACGGAA TCCCGGTGCT
951 GCCGCTACCC CCTCACGGTC GATTTTGAAG CCTTTGGATG GGACTGGATT
1001 ATCGCACCCA AAAGATATAA GGCCAATTAC TGCTCAGGAG AGTGTGAATT
1051 TGTGTTTTTA CAAAAATATC CGCATACTCA TCTTGTGCAC CAAGCAAACC
1101 CCAGAGGCTC AGCAGGCCCT TGCTGCACTC CGACAAAAAT GTCTCCCATT
1151 [?][?]TATGCTAT ATTTTAATGG CAAAGAACAA ATAATATATG GGAAAATTCC
1201 AGCCATGGTA GTAGACCGCT CTCGGTGCTC ATGAGCTTTG CATTAGGTTA
1251 GAAACTTCCC AAGTCATGGA AGGTCTTCCC CTCAATTTCG AAACTGTGAA

1301 TTCAAGCACC ACAGGCTGTA GGCCTTGAGT ATGCTCTACT AACGTAAGCA
1351 CAAGCTACAG TGTATGAACT AAAAGAGAGA ATAGATGCAA TGGTTGGCAT
1401 TCAACCACCA AAATAAACCA TACTATAGGA TGTTGTATGA TTTCCAGAGT
1451 TTTTGAAATA GATGGAGATC AAATTACATT TATGTCCATA TATGTATATT
1501 ACAACTACAA TCTAGGCAAG GAAGTGAGAG CACATCTTGT GGTCTGCTGA
1551 GTTAGGAGGG TATGATTAAA AGGTAAAGTC TTATTTCCTA ACAGTTTCAC
1601 TTAATATTTA CAGAACAATC TATATGTAGC CTTTGTAAAG TGTAGGATTG
1651 TTATCATTTA AAAACATCAT GTACACTTAT ATTTGTATTG TATACTTGGT
1701 AAGATAAAAT TCCACAAAGT AGGAATGGGG CCTCACATAC ACATTGCCAT
1751 TCCTATTATA ATTGGACAAT CCACCACGGT GCTAATGCAG TGCTCAATGG
1801 CTCCTACTGG ACCTCTCGAT AGAACACTCT ACAAAGTACG AGTCTCTCTC
1851 TCCCTTCCAG GTGCATCTCC ACACACACAG CACTAAGTGT TCAATGCATT
1901 TTCTTTAAGG AAAGAAGAAT CTTTTTTTCT AGAGGTCAAC TTTCAGTCAA
1951 CTCTAGCACA GCGGGAGTGA CTGCTGCATC TTAAAAGGCA GCCAAACAGT
2001 ATTCATTTTT TAATCTAAAT TTCAAAATCA CTGTCTGCCT TTATCACATG
2051 CCAATTTTGT GGTAAAATAA TGGAAATGAC TGGTTCTATC AATATTGTAT
2101 AAAAGACTCT CAAACAATTA CATTTATATA ATATGTATAC AATATTGTTT
2151 TGTAAATAAG TGTCTCCTTT TATATTTACT TTGGTATATT TTTACACTAA
2201 TGAAATTTCA AATCATTAAA GTACAAAGAC ATGTCATGTA TCACAAAAAA
2251 GGTGACTGCT TCTATTTCAG AGTGAATTAG CAGATTCAAT AGTGGTCTTA
2301 AAACTCTGTA TGTTAAGATT AGAAGGTTAT ATTACAATCA ATTTATGTAT
2351 TTTTTACATT ATCAACTTAT GGTTTCATGG TGGCTGTATC TATGAATGTG
2401 GCTCCCAGTC AAATTTCAAT GCCCCACCAT TTTAAAAATT ACAAGCATTA
2451 CTAAACATAC CAACATGTAT CTAAAGAAAT ACAAATATGG TATCTCAATA
2501 ACAGCTACTT TTTTATTTTA TAATTTGACA ATGAATACAT TTCTTTTATT
2551 TACTTCAGTT TTATAAATTG GAACTTTGTT TATCAAATGT ATTGTACTCA
2601 TAGCTAAATG AAATTATTTC TTACATAAAA ATGTGTAGAA ACTATAAATT
2651 AAAGTGTTTT CACATTTTTG AAACGC

### SEQUENCE ID NO. 6

1 MMQKLQMYVY IYLFMLIAAG PVDLNEGSER EENVEKEGLC NACAWRQNTR

51 YSRIEAIKIQ ILSKLRLETA PNISKDAIRQ LLPRAPPLRE LIDQYDVQRD

101 DSSDGSLEDD DYHATTETII TMPTESDFLM QADGKPKCCF FKFSSKIQYN

151 KVVKAQLWIY LRPVKTPTTV FVQILRLIKP MKDGTRYTGI RSLKLDMSPG

201 TGIWQSIDVK TVLQNWLKQP ESNLGIEIKA LDENGHDLAV TFPGPGEDGL

251 NPFLEVKVTD TPKRSRRDFG LDCDEHSTES RCCRYPLTVD FEAFGWDWII

301 APKRYKANYC SGECEFVFLQ KYPHTHLVHQ ANPRGSAGPC CTPTKMSPIN

351 MLYFNGKEQI IYGKIPAMVV DRCGCS*

### SEQUENCE ID NO. 7

hs3753 NGATTTTCTAATGCAAGTGGATCCAAAACCC

hc3753 AAATCTTGCTTCTTTAAATTTAGCTCTAAAATACAATACAATAAAGTAGTAAAGGCCCAA

hs3753 CTATCCATATATTTGAGACCCGTCGAGACTCCTACAACAGTGTTTGTGCAAATCCTGAGA

hc3753 CTCATCAAACCTATGAAAGACGGTACAAGGTATACTGGAATCCGATCTCTGAAACTTGAC

hs3753 ATGAACCCAGGCACTGGTATTTCGCAGA·NATTGATGTGAAGACACTGTTGCAAAATTGG

hs3753 CTCAAACAACCTGAATCGAACTTAGGCATTGAAATAAAAGCTTTACATGAGAATGGTCAT

hs3753 GATCTTGCTGTAACCTTCCCAGCACCAGGAAGAAGATGGGCTGAATCCCTTTTTTAAGAA

hs3753 CGTCAAGGTAACAGACACACACCAAAAAGATTCCAGAAGGGATTTTCGGTCTTGACTGGTGA

hs3753 TGAGCACTCAACAGAATCACGATCCTCTCGTTACCCCCTAACTGGTGGATTTTGAAGCCT

hs3753 TTCGCATGGGATTGGA·TATCG

hs7823 AGCGATGGTAGTAGACCGCTGTGGGTGCTC

hs7823 ATGAGATTTATATTAAGCGTTCATAACTTCCTAAAACATGGAAGCTTTTCCCCTCAACAA

hs7823 TTTTGAAGCTGTGAAATTAAGTACCACAGGCTATAGGCCTAGAGTATGCTACAGTCACTT

hs7823 AAGCATAAGCTACAGTATGTAAACTAAAAGGGGGAANGGAATATATGCAATGGTTGGCA

hs7823 TTTAACCATCCAAACAAATCATAC--CAGAAAGTTTTATGATTTCCANAGTTTTTNAGG

hs7823 CNAGAAAGGAGGAGTCAAANTTTCANTCTTATGGT

h92027 ATTTCGGCACAGGTNAAACACTTGAATTTATATTGTATGGTAGTATA

h92027 CTTGGTAAGATAAAATTCCACAAAAATAGGGATGGTGCAGCATATGCA-ATTTCCATTCC

h92027 TATTATAATTGACACAGTACATTAACAATCCATGCCAACGGTGCTAATACGATAGGCTGA

n95327 TAAATCTCAACGTTCCATTATTTTAATACTTGCAAAAACATTACTAAGTATACCAAAATA

n95327 ATTGACTCTATTATC-TG-AAATGAAG-AATAAACTGATGCTATCTCAACAATAACTGTT

n95327 ACTTTTATTTTATAATTTGATAATGAATATATTTCTGCATTTATTTACTTCTGTTTTGTA

n95327 AATTGGCATTTTGTTAATCAAATTTATTGTACT-ATGACTAAATGAAATTATTTCTTACA

n95327 T-CTAATTTGTAGAAACAGTATAAGTTATATTAAACTGTTTTCACATTTTTTTGAAAGAC

### SEQUENCE ID NO. 8

1 MQKLQLCVYI YLFMLIVAGP VDLNENSEQK ENVEKEGLCN ACTWRQNTKS
51 SRIEAIKIQI LSKLRLETAP NISKDVIRQL LPKAPPLREL IDQYDVQRDD
101 SSDGSLEDDD YHATTETIIT MPTESDFLMQ VDGKPKCCFF KFSSKIQYNK
151 VVKAQLWIYL RPVETPTTVF VQILRLIKPM KDGTRYTGIR SLKLDMNPGT
201 GIWQSIDVKT VLQNWLKQPE SNLGIEIKAL DENGHDLAVT FPGPGEDGLN
251 PFLEVKVTDT PKRSRRDFGL DCDEHSTESR CCRYPLTVDF EAFGWDWIIA
301 PKRYKANYCS GECEFVFLQK YPHTHLVHQA NPRGSAGPCC TPTKMSPINM
351 LYFNGKEQII YGKIPAMVVD RCGCS*

### SEQUENCE ID NO. 54

1 GCGGCCGCCC GGGCAGGTAT CGAAAGTTTC ACATATAAAG ATGAATAAGA
51 TCTAAGTGTA TATGTTATTG TTAATAAAGT TTTTAATTTT TCGAATGTCA
101 CATACAGCCT TTATTATTCA TAGATTTATT CCTTTTAAGA AGTAGTCAAA
151 TGAATCAGCT CACCCTTGAC TGTAACAAAA TACTGTTTGG TGACTTGTGA
201 CAGACAGGGT TTTAACCTCT GACAGCGAGA TTCATTGTGG AGCAAGAGCC
251 AATCACAGAT CCCGACGACA CTTGTCTCATCAAAGTTGGA ATATAAAAAG
301 CCACTTGGAA TACAGTATAAAAGATTCACT GGTGTGGCAA GTTGTCTCTA
351 GACTGGGCAG GCATTAACGT TTGGCTTGGC GTTACTCAAA AGCAAAAGAA
401 AAGTAAAAGG AAGAAGTAAG AACAAGGGAA AAGATTGTAT TGATTTTAAA
451 ACCATGCAAA AACTGCAAAT CTCTGTTTAT ATTTACCTAT TTATGCTGAT
501 TGTTGCTGGC CCAGTGGATC TGAATGAGAA CAGCGAGCAG AAGGAAAATG
551 TGGAAAAAGA GGGGCTGTGT AATGCATGTT TGTGGAGGGA AAACACTACA
601 TCCTCAAGAC TAGAAGCCAT AAAAATCCAA ATCCTCAGTA AACTTCGCCT
651 GGAAACAGCT CCTAACATCA GCAAAGATGC TATCAGACAA CTTTTGCCCA
701 AGGCTCCTCC ACTCCTGGAA CTGATTGATC AGTTCGATGT CCAGAGAGAT
751 GCCAGCAGTG ACGGCTCCTT GGAAGACGAT GACTACCACG CCAGGACGGA
801 AACGGTCATT ACCATGCCCA CGGAGT/GTGA GTAGTCCTGCTGGTGCAAAG
851 CAACGACTCT GCTGACTGCT GTTCTAGTGT TCATGAAAAA CCGATCTATT
901 TTCAGGCTCT TTTAACAAGC TGCTGGCTTG TATGTAAGGA GGAGGGGAAA
951 GAGCTTTTTT CAAGATTTCA TGAGAAATAG ACCAATGAGA CTGAAAGCTG
1001 CTACTTTATT TGTTTCCTTA GAGAGCTAAA AAGCTAAAAA TCAAAAATGA
1051 AATGCTTGCA TAGCATTCAT GTTATATAGT TTAGTATGAC AACTATAACA
1101 TGTTTATGTT TTCACAGCTT AATGCTACCA AGGTAAAGGA TTGGGAAACA
1151 GTATCAGCAA TGTGAAAAAT TTACATCAAA TTTCCTAATT GCATTTGGTT

1201 GCCTGAAATA TGCATTTATA ATAACAGGTT TTTTTTTTTT CATTAATAAA
1251 AGAGAAAGGA AGAAATCTGT AGAGGTTGAA GCCTATCTGG GCATTTGCTG
1301 AACACTTAGA ATGACTTCTG TTATTCAAAA CTATTTCTCA TAGGGTTTTT
1351 ATGGTCTTCA CAGAGTATCT AATTTTGAAA GCTATTAGAG TGGAAAGGAT
1401 AAAAGAATAT TCTTAATAAA CTTAATGTAT TAGTAAGAGC AATAAGGAAG
1451 TAAACACAGC ATAGTGAAAA ATCATGAGCT AATCAGCAGA AAATTCTAAG
1501 AAATAAACAT TTTAATTACA AAGTTCCACT TATACCCTGA CCATGGTACT
1551 ATTGTTGAGA GTACCTTGTC TGCACATATC TAGGAGGCAC ATGCTTAATA
1601 ACCTTCTAAA ATATTATTGT ATTCCTCATA GGAGGGAGAA CTATTACCTA
1651 TATGTAGTAC CTATGTTGTT TCTGAAAGAT AATATGTTTC ATGTATTTCT
1701 GTTGCAGTCA CTTCAAACCT ATACTCAAGG AAAGGGAGAC AGGCATCTCA
1751 ACAGAGAAGG CATGACCAGA AAGAGTTTTG TGCCATGTGT CTGCGATCTT
1801 GCTTTATACA GGGCTCTACC CACTTTAAAC TGGACTCAAA ACAGTTTCAA
1851 AATACTGCTT TTTCTTATTA AGTAACTAGT TTATAAGGCA ACAAATAAAT
1901 TTCCTTTAAG ACTGTGCTAT CAGATAATCC TGGAATAGAT TTGCCTTACT
1951 TATAAACAAT CTTGAGAAAA CAAAAAGGCA AGAAATTGCT AAGTGCTTCT
2001 GCTTACAATG ACAGCCTGGC CCTAAAGACA ATGTTTTCTA AGTTTTGAAA
2051 CAGCTTGAAT ACAACATCTA AGTTTTGGTG CTAATTACCT GCTAGTTTTT
2101 TTATTTTTTT CCTTTAAAAG GCTGTCCCAG CGTCCTAACA TAACAGATGC
2151 ACTATATTTT CTGCTAATTC CCGAGGCTCA GTTAGTTGCT CACTGTGTCT
2201 TGTCCCCAGG TAATTCAGGC CTGGGGGAAG GGTTCCTTCC TCCAGACTGA
2251 TTGGTACAGC TGCTCAGTAA GTGTAACTAC TCAGATTCCC AAAGAATTCT
2301 AAGTGGATGT TCTTCCACAG TGTCTCTTGT TCTCTCTAAT CATCATCATT
2351 TTAAAATTTC ATCCACTTTT CATTCCTTAA TAGAATTTTC CTTAGTCCAC
2401 AGTTCTCTGG AAAGGAAGTA GGCTTCTCAT AACAGCTGAA AAAACATATA
2451 CCTAAAAGAT TCTGAAAAGC TGTAATAACT GTTATACTTG ATATTTTGCT

2501 GTTATGAATG AAATGCTACA TATTTTTCCA TTTTAAAAGA CTAAATATGC
2551 ACACATTATTCCAATTAAAA AATGTTCATA GATTGATATGGAGGTGTTCG
2601 TTCATTTTTC ATAAAAATGA TCTTAGTAAC TTTTTTTCTT ATTCATTTAT
2651 AG/CTGATCTT CTAACGCAAGTGGAAGGAAA ACCCAAATGT TGCTTCTTTA
2701 AATTTAGCTC TAAGATACAA TACAATAAAC TAGTAAAGGC CCAACTGTGG
2751 ATATATCTGA GGCCTGTCAA GACTCCTGCG ACAGTGTTTG TGCAAATCCT
2801 GAGACTCATC AAACCCATGA AAGACGGTAC AAGGTATACT GGAATCCGAT
2851 CTCTGAAACT TGACATGAAC CCAGGCACTG GTATTTGGCA GAGCATTGAT
2901 GTGAAGACAG TGTTGCAGAA CTGGCTCAAA CAACCTGAAT CCAACTTAGG
2951 CATTGAAATC AAAGCTTTAG ATGAGAATGG CCATGATCTT GCTGTAACCT
3001 TCCCAGAACC AGGAGAAGAT GGACTG/GTAA GTGATTACTGAAAATAACAT
3051 GCTAAAAACC TTGTTATGTG TTTATTCATA ATGTGAATGA ATAGTAGTGA
3101 AAAATAACTA CCAGTTTCCT GTGCTTATAA GCCAGACAAA GGCACCTTAC
3151 CCCAGTGGTA GCCCTGTACT CAATAAAAGT AGGTGTCCCA TTTCACATCC
3201 TATGAAACAC TCTCTTGATA CTTTGACTTT GCATGAGGAT TTAAAAGAAA
3251 AAAAGTTATA CCATGGTCCT TAAGTTTTTA GGGAATTCTT TGGAATTGAG
3301 AATGAAATAT AAAATGCTTT CCGTTGATGT GCTACATGAT TATATAAATA
3351 AAAACATGAA GTCTTCACAG TGGATTCTAG TACTCACCCA ACAACACATT
3401 TTTTCCCCCA GAAGAGTGAC CAATTTGTTA AAATTCTTTT GCTTAATAAG
3451 GCAGAAAAAT GAACTCTACA AGTTATAATT AAAATAAAAT GCTTTTACTT
3501 ATAGAAATTA ACTAGATATA TGTTCAGGTT TATATACTAT TAAATATACT
3551 ATATTTAAGA TCTCTCATGA TAAATATGTT CCTTGTTTTA TAGACTATTG
3601 ATGCACTGAT GTATATGTGG ATTACTTTGT GAATTACCCC TGGTAAAATT
3651 AAAAATTTCA GGCTAGTTAA CTTGTACTAC TTAGCTATTT TCTGAACTGT
3701 CTTACTGTTC TTTAACAGGA GTTAACTTAG GTAATGTCAA CTAATTTAAT

3751 ATAAAGTCAA ACAGAAAATA ATGCCTTATA TATTATAAAA ATTAATAAAA
3801 AACCATTTTA AAATCTAGTA TAAGTTTAGA GCTACTCACT CTTCTGGCTT
3851 ATCTATGCTT GTATTTACTT CTGTTTTCAA AAAATTTTTT AATGTGACCA
3901 TACCTTTTAT TTCCAGTTAT TGATATAATT TACAACAAAA GATTATACTT
3951 GCAAGCTTTA TAGTTTTTAA ATGGTCTTAT TTGTAGTGAA TATCATATCT
4001 AAATGATATC TAAATGTAAA GTAAATCATA CCTAAATGAA AACATATTCT
4051 TTAAGTCATT ATAAAATTTT CCAGGTGATC AATTTTTCTT TAAATATACT
4101 ACATAAAATG TTATTGACTC CCAAAATGAT GTTATTTTGT ATAATCTTAA
4151 ATACCAATAA TTACCAGGTC TATTTTGGTT TTAGTGTAGG ATAAAAAAGA
4201 ATGTGTTCTT TTTTCTAGGT AGCATTTTAA TGATCAAAGT TGGTGACGTG
4251 ACAGAGGTCT TAAGTATTAT TAAACAGATG ATTAATAAGA TGTATTCCTC
4301 AGACTTTTCC ATATAAAAGG AAAAATGTCT CAAATTCATG AAAAGATTGG
4351 TACAGGAGGA GGATTAGCAA ATTGTAGTTT AAATATCTGA ATGGAAACAC
4401 TTTTTAGTGA AAGAATAAAG GGAATATCAT TGTATCTTCT TCTGAGTCTG
4451 TGCCTCTCTC TCTTGGAGTT AGTCTTTCCA ACCCTATATA CTTACCACTA
4501 TCTTCATCCC TCTACCTTCC TTTTTCCCAT TACATCTGTG CAGTACTGGG
4551 TGGCAACTAT TGTGTTTCGG TGTTAATATC CAAGTTTCCC TGAATAAGAC
4601 CAAGTGAATG GAGGATGAAT GAGTATACCT ATCCCTCCAG GGGTCATCAG
4651 ACATATTTAG CCACCATATT TAATCAATAA GCAGGAAGAC ATAAGCTAGC
4701 CTTGTCCTTC TTCTTTCCTC CCTGCTCCTT TCTCTTCTCT TCCCCCTCTC
4751 CCTTTACTGT CATCCATCAG TATTTTCAGA GCATCTATTA TGTGTCAGGC
4801 ATTCAGATAC TCAAACGGAG GAAAACAAGA ATAAACAAGA CAAAGATCTG
4851 ACCACAGGGG AATCCCTATG GCTACTGTAG ACTTTTGAGC CATAAAGGAA
4901 GAATCAAGCC TAGTGTAAAT GAAAATTCCT TAATGCTGTG CCTTTTAAAA
4951 AGAAATGTGA CATAAGCAAA ATGATTAGTTTCTTTCTTTA ATAATGAGTC
5001 CTTGAGGTAG GAGAGTGTTT TGGGATCTATTATTAACTCT TCTTTCCTTT

5051 CCATACAG/AC TCCTTTTTTA GAAGTCAAGG TAACAGACAC ACCAAAAAGA
5101 TCTAGGAGAG ATTTTGGGCT TGATTGTGAT GAACACTCCA CAGAATCTCG
5151 ATGCTGTCGT TACCCTCTAA CTGTGGATTT TGAAGCTTTT GGATGGGATT
5201 GGATTATTGC ACCTAAAAGA TATAAGGCCA ATTACTGCTC TGGAGAATGT
5251 G[?][?]TTTGTAT TTTTGCAAAA GTATCCTCAT ACCCATCTTG TGCACCAAGC
5301 AAACCCCAGA GGTTCAGCCG GCCCCTGCTG TACTCCTACA AAGATGTCTC
5351 CAATTAATAT GCTATATTTT AATGGCGAAG GACAAATAAT ATACGGGAAG
5401 ATTCCAGCCA TGGTAGTAGA TCGCTGTGGGTGTTCATGAG GTCTATATTT
5451 GGTTCATAGC TTCCTCAAAC ATGGAAGGTC TTCCCCTCAA CAATTTTGAA
5501 ACTGTGAAAT TATGTACCAC AGGCTATAAG CCTAGAGTAT GCTACAGTCA
5551 CTTAAGCACA AGCTACAGTA TATGAGCTAA AAAGAGAGAA TATATGCAAT
5601 GGTTGGCATT TAACCATCCA AACAAATCGT ATAATAAAAA GTTTTATGAT
5651 TTCCAGAGTT TTTGAACTAG GAGATCAAAT TCCATTTATG TTGAAATATA
5701 TTACAACACA TGCAGGTGAA TGAAAGCAAT TCTCCTTGTC TTCTGGTGAA
5751 TTAAAGGAGT ATGCTTTAAA ATCTATTTCT CTACAGTTTC

## Claims

1. A transgenic male non-human mammal having a phenotype **characterized by** muscular hyperplasia, said phenotype being conferred by a transgene contained in the somatic and germ cells of the mammal, the transgene encoding a myostatin protein having a dominant negative mutation located on the Y chromosome.

2. The transgenic mammal of claim 1 wherein the transgene is located to be under the control of a promoter such that the gene is expressed in a specific tissue, optionally, wherein the tissue is skeletal muscle, and wherein the promoter can be of a myosin gene.

3. A transgenic non-human mammal having a phenotype **characterized by** muscular hyperplasia, said phenotype being conferred by a transgene having a sequence antisense to that encoding a myostatin protein of the mammal, said transgene being located on the Y chromosome of the mammal.

4. The transgenic mammal of claim 3 wherein the mammal is bovine, and/or wherein the transgene further comprises a sequence which when transcribed obtains an RNA having ribozyme activity.

5. A transgenic non-human mammal having a phenotype **characterized by** muscular hyperplasia, said phenotype being inducible and being conferred by a nucleic acid molecule comprising a sequence of a naturally occurring mutation of a coding portion of the myostatin gene, flanked by loxP sites and a Cre transgene under the dependence of an inducible promoter.

6. A transgenic non-human male mammal having a phenotype **characterized by** muscular hyperplasia, said phenotype being conferred by a myostatin gene flanked by loxP sites and a Cre transgene located on the Y chromosome.

7. An in vitro method for creating a cell for use in creating a transgenic male non-human mammal having a phenotype **characterized by** muscular hyperplasia, the method comprising the step of introducing into the Y chromosome of the cell a polynucleotide molecule encoding a myostatin protein having a dominant negative mutation.

8. The method of claim 7, wherein the mammal is bovine, and optionally, comprising the further step of creating said dominant mutation by mutating the proteolytic processing site of a polynucleotide molecule encoding a myostatin protein.

9. The method of claim 7 or 8 wherein the polynucleotide molecule encoding a myostatin protein having a dominant negative mutation is introduced into the Y chromosome so as to be put under the control of a promoter which is stronger than its natural promoter or a tissue specific promoter other than its natural promoter, and wherein the promoter can be the CMV promoter or the promoter of the myosin gene.

10. An in vitro method for creating a cell for use in creating a transgenic male non-human mammal having a phenotype **characterized by** muscular hyperplasia, the method comprising the step of introducing into the Y chromosome of a totipotent cell line of said mammal, a polynucleotide molecule encoding a myostatin protein having a dominant negative mutation.

11. The method of claim 10 wherein the step of introducing a polynucleotide molecule into the Y chromosome is accomplished by means of homologous recombination, and/or optionally, wherein the mammal is bovine, and optionally, comprising the further step of creating said dominant mutation by mutating the proteolytic processing site of a polynucleotide molecule encoding a myostatin protein.

12. An in vitro method for creating a cell for use in creating a transgenic male non-human mammal having a phenotype **characterized by** muscular hyperplasia, the method comprising the step of introducing into the Y chromosome of the genome of the cell, a polynucleotide molecule comprising a sequence antisense to a sequence encoding a myostatin protein.

13. The method of claim 12 wherein the mammal is bovine, and optionally, wherein the sequence of said polynucleotide molecule comprises a sequence which is antisense to SEQ ID NO:1.

14. The method of claim 11 or 12, wherein said polynucleotide molecule further encodes an RNA sequence having ribozyme activity.

15. The method of claim 7, 8, 9, 10, 11, 12 or 13, wherein the polynucleotide molecule encoding a myostatin protein having a dominant negative mutation is introduced into the Y chromosome so as to be put under the control of a promoter the expression of which is inducible.

16. The method of claim 9 or 15, wherein the promoter undergoes post-natal expression in skeletal muscle.

17. An in vitro method for creating a cell for use in creating a transgenic male non-human mammal having a phenotype **characterized by** muscular hyperplasia, the method comprising the step of introducing into the Y chromosome of a totipotent cell line of said mammal, a polynucleotide molecule comprising a sequence antisense to a sequence encoding a myostatin protein.

18. The method of claim 17 wherein the mammal is bovine, and optionally, wherein the sequence of said polynucleotide molecule comprises a sequence which is antisense to SEQ ID NO:1.

19. The method of claim 17 or 18, wherein said polynucleotide molecule encodes an RNA sequence having ribozyme activity.

20. The method of claim 17, 18 or 19, wherein the polynucleotide molecule is introduced into the Y chromosome so as to be put under the control of a promoter the expression of which is inducible, wherein the promoter optionally undergoes post-natal expression in skeletal muscle.

21. A transgenic non-human male mammal having a phenotype **characterized by** muscular hyperplasia, said phenotype being conferred by a transgene contained in the somatic and germ cells of the mammal, the transgene comprising a sequence of a naturally occurring mutation of a coding portion of the myostatin gene and being under the dependence of an inducible promoter.

22. The transgenic mammal of claim 21, wherein said dominant negative mutation is located on the Y chromosome, and/or wherein the mutation is known to naturally occur and which when present in both alleles of a mammal results in muscular hyperplasia.

23. The transgenic mammal of claim 22 wherein the mammal is bovine and the mutation is selected from the group of mutations resulting from: (a) deletion of 11 nucleotides beginning at nucleotide 821 of the coding portion of SEQ ID NO:1; (b) deletion of 7 nucleotides beginning at nucleotide 419 of the coding sequence and insertion of the sequence AAGCATACAA in place thereof; (c) deletion of nucleotide 204 of the coding sequence and insertion of T in place thereof; (d) deletion of nucleotide 226 of the coding sequence and insertion of T in place thereof; and (e) deletion of nucleotide 313 of the coding sequence and insertion of A in place thereof; and combinations thereof, and optionally, wherein the mutation results from deletion of 11 nucleotides beginning at nucleotide 821 of the coding portion of SEQ ID NO:1.

24. A transgenic non-human male mammal having a phenotype **characterized by** muscular hyperplasia, said phenotype being conferred by a transgene located on the Y chromosome and contained in the somatic and germ cells of the mammal, the transgene encoding a polynucleotide molecule comprising a sequence antisense to a sequence encoding a myostatin protein.

25. The transgenic mammal of claim 24, wherein the mammal is bovine, and optionally, wherein the sequence of said polynucleotide molecule comprises a sequence which is antisense to SEQ ID NO:1, and/or wherein said polynucleotide molecule encodes an RNA sequence having ribozyme activity.

26. The transgenic mammal of claim 24 or 25, wherein the polynucleotide molecule is located on the Y chromosome so as to be under the control of a promoter the expression of which is inducible.

27. The transgenic mammal of claim 24, 25 or 26, wherein the polynucleotide molecule is under the control of a promoter which undergoes post-natal expression in skeletal muscle.

28. The transgenic non-human mammal of claim 5 or claim 21, wherein mutation is selected from the group of mutations resulting from: (a) deletion of 11 nucleotides beginning at nucleotide 821 of the coding portion of SEQ ID NO:1; (b) deletion of 7 nucleotides beginning at nucleotide 419 of the coding sequence and insertion of the sequence AAGCATACAA in place thereof; (c) deletion of nucleotide 204 of the coding sequence and insertion of T in place thereof; (d) deletion of nucleotide 226 of the coding sequence and insertion of T in place thereof; and (e) deletion of nucleotide 313 of the coding sequence and insertion of A in place thereof; and combinations thereof, preferably wherein the mutation results from deletion of 11 nucleotides beginning at nucleotide 821 of the coding portion of SEQ ID NO:1.

## Patentansprüche

1. Transgenes männliches, nicht menschliches Säugetier mit einem Phänotyp, der durch eine muskuläre Hyperplasie charakterisiert ist, wobei der Phänotyp durch ein Transgen verliehen wird, das in den Körper- und Keimzellen des Säugetiers enthalten ist, wobei das Transgen, das ein Myostatin-Protein codiert, eine dominant-negative Mutation aufweist, die auf dem Y-Chromosom lokalisiert ist.

2. Transgenes Säugetier nach Anspruch 1, wobei das Transgen so liegt, dass es unter der Kontrolle eines Promotors steht, so dass das Gen in einem spezifischen Gewebe exprimiert wird, wobei das Gewebe gegebenenfalls Skelettmuskulatur ist, und wobei der Promotor von einem Myosin-Gen sein kann.

3. Transgenes nicht menschliches Säugetier mit einem Phänotyp, der durch eine muskuläre Hyperplasie charakterisiert ist, wobei der Phänotyp durch ein Transgen verliehen wird, das eine Sequenz aufweist, die eine Antisense-Sequenz zu der ist, die ein Myostatin-Protein des Säugetiers codiert, wobei das Transgen auf dem Y-Chromosom des Säugetiers lokalisiert ist.

4. Transgenes Säugetier nach Anspruch 3, wobei das Säugetier ein Rind ist, und/oder wobei das Transgen ferner eine Sequenz umfasst, die, wenn transkribiert, zu einer RNA mit Ribozymaktivität führt.

5. Transgenes nicht menschliches Säugetier mit einem Phänotyp, der durch eine muskuläre Hyperplasie charakterisiert ist, wobei der Phänotyp induzierbar ist und durch ein Nucleinsäuremolekül verliehen wird, das eine Sequenz einer natürlich auftretenden Mutation eines codierenden Teils des Myostatin-Gens umfasst, flankiert von loxP-Stellen, und ein Cre-Transgen unter der Abhängigkeit eines induzierbaren Promotors.

6. Transgenes nicht menschliches, männliches Säugetier mit einem Phänotyp, der durch eine muskuläre Hyperplasie charakterisiert ist, wobei der Phänotyp durch ein Myostatin-Gen, flankiert von loxP-Stellen, und ein Cre-Transgen, lokalisiert auf dem Y-Chromosom, verliehen wird.

7. In vitro-Verfahren zur Herstellung einer Zelle zur Verwendung in der Erzeugung eines transgenen männlichen, nicht-menschlichen Säugetiers mit einem Phänotyp, der durch eine muskuläre Hyperplasie charakterisiert ist, wobei das Verfahren den Schritt des Einführens eines Polynucleotidmoleküls mit einer dominant-negativen Mutation, das ein Myostatin-Protein codiert, in das Y-Chromosom der Zelle umfasst.

8. Verfahren nach Anspruch 7, wobei das Säugetier ein Rind ist, und gegebenenfalls den weiteren Schritt umfassend, die dominante Mutation zu erzeugen, indem die Stelle der proteolytischen Prozessierung eines Polynucleotidmoleküls, das ein Myostatin-Protein codiert, mutiert wird.

9. Verfahren nach Anspruch 7 oder 8, wobei das Polynucleotidmolekül mit einer dominant-negativen Mutation, das ein Myostatin-Protein codiert, so in das Y-Chromosom eingeführt wird, dass es unter die Kontrolle eines Promotors gestellt ist, der stärker als sein natürlicher Promotor ist, oder eines Gewebespezifischen Promotors, der nicht sein natürlicher Promotor ist, und wobei der Promotor der CMV-Promotor oder der Promotor des Myosin-Gens sein kann.

10. In vitro-Verfahren zur Herstellung einer Zelle zur Verwendung in der Erzeugung eines transgenen männlichen nicht menschlichen Säugetiers mit einem Phänotyp der durch eine muskuläre Hyperplasie charakterisiert ist, wobei das Verfahren den Schritt des Einführens eines Polynucleotidmoleküls mit einer dominant-negativen Mutation, das ein Myostatin-Protein codiert, in das Y-Chromosom einer totipotenten Zelllinie des Säugetiers umfasst.

11. Verfahren nach Anspruch 10, wobei der Schritt des Einführens eines Polynucleotidmoleküls in das Y-Chromosom durch homologe Rekombination durchgeführt wird, wobei das Säugetier ein Rind ist, und gegebenenfalls den weiteren Schritt umfassend, die dominante Mutation zu erzeugen, indem die Stelle der proteolytischen Prozessierung eines Polynucleotidmoleküls, das ein Myostatin-Protein codiert, mutiert wird.

12. In vitro-Verfahren zur Herstellung einer Zelle zur Verwendung in der Erzeugung eines männlichen nicht menschlichen Säugetiers mit einem Phänotyp, der durch eine muskuläre Hyperplasie charakterisiert ist, wobei das Verfahren den Schritt des Einführens eines Polynucleotidmoleküls, das eine Sequenz aufweist, die eine Antisense-Sequenz zu einer das Myostatin-Protein codierenden Sequenz ist, in das Y-Chromosom des Genoms der Zelle umfasst.

13. Verfahren nach Anspruch 12, wobei das Säugetier ein Rind ist, und gegebenenfalls, wobei die Sequenz des Polynucleotidmoleküls eine Sequenz umfasst, die eine Antisense-Sequenz zu SEQ ID NO:1 ist.

14. Verfahren nach Anspruch 11 oder 12, wobei das Polynucleotidmolekül ferner eine RNA-Sequenz codiert, die Ribozymaktivität besitzt.

15. Verfahren nach Anspruch 7, 8, 9, 10, 11, 12 oder 13, wobei das Polynucleotidmolekül mit einer dominant-negativen Mutation, das ein Myostatin-Protein codiert, so in das Y-Chromosom eingeführt ist, dass es unter der Kontrolle eines Promotors steht, dessen Expression induzierbar ist.

16. Verfahren nach Anspruch 9 oder 15, wobei der Promotor post-natal in Skelettmuskulatur exprimiert wird.

17. In vitro-Verfahren zur Herstellung einer Zelle zur Verwendung in der Erzeugung eines männlichen nicht menschlichen Säugetiers mit einem Phänotyp, der durch eine muskuläre Hyperplasie charakterisiert ist, wobei das Verfahren den Schritt des Einführens eines Polynucleotidmoleküls, das eine Sequenz aufweist, die eine Antisense-Sequenz zu einer ein Myostatin-Protein codierenden Sequenz ist, in das Y-Chromosom einer totipotenten Zelllinie des Säugetiers umfasst.

18. Verfahren nach Anspruch 17, wobei das Säugetier ein Rind ist, und, gegebenenfalls, wobei die Sequenz des Polynucleotidmoleküls eine Sequenz umfasst, die eine Antisense-Sequenz zu SEQ ID NO:1 ist.

19. Verfahren nach Anspruch 17 oder 18, wobei das Polynucleotidmolekül eine RNA-Sequenz codiert, die Ribozymaktivität besitzt.

20. Verfahren nach Anspruch 17, 18 oder 19, wobei das Polynucleotidmolekül so in das Y-Chromosom eingeführt ist, dass es unter der Kontrolle eines Promotors steht, dessen Expression induzierbar ist, wobei der Promotor gegebenenfalls post-natal in Skelettmuskulatur exprimiert wird.

21. Transgenes nicht menschliches, männliches Säugetier mit einem Phänotyp, der durch eine muskuläre Hyperplasie charakterisiert ist, wobei der Phänotyp durch ein Transgen verliehen wird, das in den Körper- und Keimzellen des Säugetiers enthalten ist, wobei das Transgen die Sequenz einer natürlich vorkommenden Mutation eines codierenden Teils des Myostatin-Gens umfasst und unter dem Einfluss eines induzierbaren Promotors steht.

22. Transgenes Säugetier nach Anspruch 21, wobei die dominant-negative Mutation auf dem Y-Chromosom lokalisiert ist, und/oder von der Mutation bekannt ist, dass sie natürlich vorkommt, und die, wenn sie in beiden Allelen eines Säugetiers vorkommt, zu muskulärer Hyperplasie führt.

23. Transgenes Säugetier nach Anspruch 22, wobei das Säugetier ein Rind ist und die Mutation ausgewählt ist aus der Gruppe von Mutationen, die resultieren von: (a) einer Deletion von 11 Nucleotiden beginnend bei Nucleotid 821 des codierenden Teils der SEQ ID NO: 1; (b) einer Deletion von 7 Nucleotiden beginnend bei Nucleotid 419 der codierenden Sequenz und einer Insertion der Sequenz AAGCATACAA anstelle davon; (c) einer Deletion von Nucleotid 204 der codierenden Sequenz und einer Insertion von T anstelle davon; (d) einer Deletion von Nucleotid 226 der codierenden Sequenz und einer Insertion von T anstelle davon; und (e) einer Deletion von Nucleotid 313 der codierenden Sequenz und einer Insertion von A anstelle davon; und Kombinationen davon, und gegebenenfalls, wobei die Mutation aus der Deletion von 11 Nucleotiden beginnend bei Nucleotid 821 des codierenden Teils der SEQ ID NO:1 resultiert.

24. Transgenes nicht menschliches, männliches Säugetier mit einem Phänotyp, der durch eine muskuläre Hyperplasie charakterisiert ist, wobei der Phänotyp durch ein Transgen verliehen wird, das auf dem Y-Chromosom lokalisiert ist und das in den Körper- und Keimzellen des Säugetiers enthalten ist, wobei das Transgen ein Polynucleotidmolekül codiert, das eine Sequenz aufweist, die eine Antisense-Sequenz zu einer ein Myostatin-Protein codierenden Sequenz umfasst.

25. Transgenes Säugetier nach Anspruch 24, wobei das Säugetier ein Rind ist, und gegebenenfalls, wobei die Sequenz des Polynucleotidmoleküls eine Sequenz umfasst, die eine Antisense-Sequenz zu SEQ ID NO: 1 ist, und/oder wobei das Polynucleotidmolekül eine RNA-Sequenz codiert, die Ribozymaktivität besitzt.

26. Transgenes Säugetier nach Anspruch 24 oder 25, wobei das Polynucleotidmolekül so auf dem Y-Chromosom lokalisiert ist, dass es unter der Kontrolle eines Promotors steht, dessen Expression induzierbar ist.

27. Transgenes Säugetier nach Anspruch 24, 25 oder 26, wobei das Polynucleotidmolekül unter der Kontrolle eines Promotors steht, der post-natal in Skelettmuskulatur exprimiert wird.

28. Transgenes nicht menschliches Säugetier nach Anspruch 5 oder Anspruch 21, wobei die Mutation ausgewählt ist aus der Gruppe von Mutationen, die resultieren von: (a) einer Deletion von 11 Nucleotiden beginnend bei Nucleotid 821 des codierenden Teils von SEQ ID NO: 1; (b) einer Deletion von 7 Nucleotiden beginnend bei Nucleotid 419 der codierenden Sequenz und einer Insertion der Sequenz AAGCATACAA anstelle davon; (c) einer Deletion von Nucleotid 204 der codierenden Sequenz und einer Insertion von T anstelle davon; (d) einer Deletion von Nucleotid 226 der codierenden Sequenz und einer Insertion von T anstelle davon; und (e) einer Deletion von Nucleotid 313 der codierenden Sequenz und einer Insertion von A anstelle davon; und Kombinationen davon, wobei vorzugsweise die Mutation aus der Deletion von 11 Nucleotiden beginnend bei Nucleotid 821 des codierenden Teils der SEQ ID NO:1 resultiert.

## Revendications

1. Mammifère transgénique mâle non humain ayant un phénotype **caractérisé par** une hyperplasie musculaire, ledit phénotype étant conféré par un transgène contenu dans les cellules somatiques et germinales du mammifère, le transgène codant pour une protéine myostatine ayant une mutation dominante négative localisée sur le chromosome Y.

2. Mammifère transgénique selon la revendication 1, chez lequel le transgène est localisé pour être sous le contrôle d'un promoteur de telle manière que le gène est exprimé dans un tissu spécifique, optionnellement, où le tissu est le muscle squelettique, et où le promoteur peut être d'un gène de la myosine.

3. Mammifère transgénique non humain ayant un phénotype **caractérisé par** une hyperplasie musculaire, ledit phénotype étant conféré par un transgène ayant une séquence antisens à celle codant pour une protéine myostatine du mammifère, ledit transgène étant localisé sur le chromosome Y du mammifère.

4. Mammifère transgénique selon la revendication 3, chez lequel le mammifère est un bovin, et/ou chez lequel le transgène comprend en outre une séquence qui lorsqu'elle est transcrite obtient un ARN ayant une activité de ribozyme.

5. Mammifère transgénique non humain ayant un phénotype **caractérisé par** une hyperplasie musculaire, ledit phénotype étant inductible et étant conféré par une molécule d'acide nucléique comprenant une séquence d'une mutation existant à l'état naturel d'une partie codante du gène de la myostatine, flanquée de sites loxP et un transgène Cre sous la dépendance d'un promoteur inductible.

6. Mammifère transgénique mâle non humain ayant un phénotype **caractérisé par** une hyperplasie musculaire, ledit phénotype étant conféré par un gène de la myostatine flanqué de sites loxP et un transgène Cre localisés sur le chromosome Y.

7. Procédé *in vitro* de création d'une cellule pour une utilisation dans la création d'un mammifère transgénique mâle non humain ayant un phénotype **caractérisé par** une hyperplasie musculaire, le procédé comprenant l'étape d'introduction dans le chromosome Y de la cellule d'une molécule polynucléotidique codant pour une protéine myostatine ayant une mutation dominante négative.

8. Procédé selon la revendication 7, dans lequel le mammifère est un bovin, et optionnellement, comprenant en outre l'étape de création de ladite mutation dominante en mutant le site de traitement protéolytique d'une molécule polynucléotidique codant pour une protéine myostatine.

9. Procédé selon la revendication 7 ou 8, dans lequel la molécule polynucléotidique codant pour une protéine myostatine ayant une mutation dominante négative est introduite dans le chromosome Y de manière à être placée sous le contrôle d'un promoteur qui est plus fort que son promoteur naturel ou d'un promoteur tissu-spécifique autre que son promoteur naturel, et dans lequel le promoteur peut être le promoteur du CMV ou le promoteur du gène de la myosine.

10. Procédé *in vitro* de création d'une cellule pour une utilisation dans la création d'un mammifère transgénique mâle non humain ayant un phénotype **caractérisé par** une hyperplasie musculaire, le procédé comprenant l'étape d'introduction dans le chromosome Y d'une lignée cellulaire totipotente dudit mammifère, une molécule polynucléotidique codant pour une protéine myostatine ayant une mutation dominante négative.

11. Procédé selon la revendication 10, dans lequel l'étape d'introduction d'une molécule polynucléotidique dans le chromosome Y est accomplie au moyen d'une recombinaison homologue, et/ou optionnellement, dans lequel le mammifère est un bovin, et optionnellement, comprenant en outre l'étape de création de ladite mutation dominante en mutant le site de traitement protéolytique d'une molécule polynucléotidique codant pour une protéine myostatine.

12. Procédé *in vitro* de création d'une cellule pour une utilisation dans la création d'un mammifère transgénique mâle non humain ayant un phénotype **caractérisé par** une hyperplasie musculaire, le procédé comprenant l'étape d'introduction dans le chromosome Y du génome de la cellule, d'une molécule polynucléotidique comprenant une séquence antisens à une séquence codant pour une protéine myostatine.

13. Procédé selon la revendication 12, dans lequel le mammifère est un bovin, et optionnellement, dans lequel la séquence de ladite molécule polynucléotidique comprend une séquence qui est antisens à SEQ ID NO : 1.

14. Procédé selon la revendication 11 ou 12, dans lequel ladite molécule polynucléotidique code en outre pour une séquence d'ARN ayant une activité de ribozyme.

15. Procédé selon la revendication 7, 8, 9, 10, 11, 12 ou 13, dans lequel la molécule polynucléotidique codant pour une protéine myostatine ayant une mutation dominante négative est introduite dans le chromosome Y de manière à être ainsi placée sous le contrôle d'un promoteur dont l'expression est inductible.

16. Procédé selon la revendication 9 ou 15, dans lequel le promoteur subit une expression post-natale dans le muscle squelettique.

17. Procédé *in vitro* de création d'une cellule pour une utilisation dans la création d'un mammifère transgénique mâle non humain ayant un phénotype **caractérisé par** une hyperplasie musculaire, le procédé comprenant l'étape d'introduction dans le chromosome Y d'une lignée cellulaire totipotente dudit mammifère, une molécule polynucléotidique comprenant une séquence antisens à une séquence codant pour une protéine myostatine.

18. Procédé selon la revendication 17, dans lequel le mammifère est un bovin, et optionnellement, dans lequel la séquence de ladite molécule polynucléotidique comprend une séquence qui est antisens à SEQ ID NO : 1.

19. Procédé selon la revendication 17 ou 18, dans lequel ladite molécule polynucléotidique code pour une séquence d'ARN ayant une activité de ribozyme.

20. Procédé selon la revendication 17, 18 ou 19, dans lequel la molécule polynucléotidique est introduite dans le chromosome Y de manière à être ainsi placée sous le contrôle d'un promoteur dont l'expression est inductible, où le promoteur subit optionnellement une expression post-natale dans le muscle squelettique.

21. Mammifère transgénique mâle non humain ayant un phénotype **caractérisé par** une hyperplasie musculaire, ledit phénotype étant conféré par un transgène contenu dans les cellules somatiques et germinales du mammifère, le transgène comprenant une séquence d'une mutation existant à l'état naturel d'une partie codante du gène de la myostatine et étant sous la dépendance d'un promoteur inductible.

22. Mammifère transgénique selon la revendication 21, chez lequel ladite mutation dominante négative est localisée sur le chromosome Y, et/ou chez lequel la mutation est connue pour exister à l'état naturel et qui, lorsqu'elle est présente dans les deux allèles d'un mammifère, entraîne une hyperplasie musculaire.

23. Mammifère transgénique selon la revendication 22, où le mammifère est un bovin et la mutation est choisie dans le groupe de mutations résultant : (a) d'une délétion de 11 nucléotides débutant au nucléotide 821 de la partie codante de SEQ ID NO : 1 ; (b) d'une délétion de 7 nucléotides débutant au nucléotide 419 de la séquence codante et d'une insertion de la séquence AAGCATACAA à la place de ceux-ci ; (c) d'une délétion du nucléotide 204 de la séquence codante et d'une insertion de T à la place de celui-ci ; (d) d'une délétion du nucléotide 226 de la séquence codante et d'une insertion de T à la place de celui-ci ; et (e) d'une délétion du nucléotide 313 de la séquence codante et d'une insertion de A à la place de celui-ci ; et de combinaisons de celles-ci, et optionnellement, où la mutation résulte de la délétion de 11 nucléotides débutant au nucléotide 821 de la partie codante de SEQ ID NO : 1.

24. Mammifère transgénique mâle non humain ayant un phénotype **caractérisé par** une hyperplasie musculaire, ledit phénotype étant conféré par un transgène localisé sur le chromosome Y et contenu dans les cellules somatiques et germinales du mammifère, le transgène codant pour une molécule polynucléotidique comprenant une séquence antisens à une séquence codant pour une protéine myostatine.

25. Mammifère transgénique selon la revendication 24, où le mammifère est un bovin, et optionnellement, chez lequel la séquence de ladite molécule polynucléotidique comprend une séquence qui est antisens à SEQ ID NO : 1, et/ou chez lequel ladite molécule polynucléotidique code pour une séquence d'ARN ayant une activité de ribozyme.

26. Mammifère transgénique selon la revendication 24 ou 25, chez lequel la molécule polynucléotidique est localisée sur le chromosome Y de manière à être placée sous le contrôle d'un promoteur dont l'expression est inductible.

27. Mammifère transgénique selon la revendication 24, 25 ou 26, chez lequel la molécule polynucléotidique est sous le contrôle d'un promoteur qui subit une expression post-natale dans le muscle squelettique.

28. Mammifère transgénique non humain selon la revendication 5 ou la revendication 21, chez lequel la mutation est choisie dans le groupe de mutations résultant : (a) d'une délétion de 11 nucléotides débutant au nucléotide 821 de la partie codante de SEQ ID NO : 1 ; (b) d'une délétion de 7 nucléotides débutant au nucléotide 419 de la séquence codante et d'une insertion de la séquence AAGCATACAA à la place de ceux-ci ; (c) d'une délétion du nucléotide 204 de la séquence codante et d'une insertion de T à la place de celui-ci ; (d) d'une délétion du nucléotide 226 de la séquence codante et d'une insertion de T à la place de celui-ci ; et (e) d'une délétion du nucléotide 313 de la séquence codante et d'une insertion de A à la place de celui-ci ; et de combinaisons de celles-ci, de préférence, où la mutation résulte de la délétion de 11 nucléotides débutant au nucléotide 821 de la partie codante de SEQ ID NO : 1.
